# FASCICULE DE BREVET EUROPEEN

(11) **EP 4 302 314 B1**
(45) Date de publication et mention de la délivrance du brevet: **02.04.2025**
(21) Numéro de dépôt: 22710134.2
(22) Date de dépôt: 24.02.2022
(51) Int. Cl.: A61L 2/238, G21F 9/00, G21F 9/28, G21F 9/04, G21F 9/02, A61L 2/18

(54) **PROCEDE DE DECONTAMINATION DE SURFACES OU DE MILIEUX GAZEUX UTILISANT UN GEL FERROMAGNETIQUE**
VERFAHREN ZUR DEKONTAMINATION VON OBERFLÄCHEN ODER GAS MITTELS EINES FERROMAGNETISCHEN GELS
PROCESS FOR DECONTAMINATION OF SURFACES OR GASES USING A FERROMAGNETIC GEL

(30) Priorité: 03.03.2021 FR 2102063
(43) Date de publication de la demande: 10.01.2024
(73) Titulaire: Commissariat à l'Energie Atomique et aux Energies Alternatives, 75015 Paris (FR)
(72) Inventeur: GOSSARD, Alban, 84000 AVIGNON (FR); FRANCES, Fabien, 30760 SAINT-JULIEN DE PEYROLAS (FR); TURC, Hubert-Alexandre, 30133 LES ANGLES (FR)
(74) Mandataire: Brevalex
(86) Numéro de dépôt international: PCT/FR2022/050337
(87) Numéro de publication internationale: WO 2022/184996

(56) Documents cités:
- FR-A1- 2 984 170
- US-A1- 2004 101 564

## Description

### DOMAINE TECHNIQUE

La présente invention a pour objet un procédé de décontamination de surfaces ou de milieux gazeux, qui utilise un gel de décontamination ferromagnétique.

Les gels utilisés dans le procédé selon l'invention sont des gels aimantables, c'est-à-dire qu'ils sont sensibles au champ magnétique créé par un aimant.

Les gels utilisés dans le procédé selon l'invention sont en outre, généralement des gels aspirables.

La présente invention a trait, tout d'abord, à un procédé de décontamination de surfaces utilisant un gel de décontamination ferromagnétique.

Le procédé de décontamination de surfaces selon l'invention permet la décontamination de toutes sortes de surfaces telles que les surfaces en matériaux métalliques, plastiques, minéraux, comme les matériaux vitreux.

Le procédé de décontamination de surfaces selon l'invention s'applique notamment, entre autres, à la décontamination de surfaces de matériaux poreux tels que les matériaux cimentaires comme les mortiers et les bétons ; les briques ; les plâtres ; et la pierre naturelle ou artificielle.

Le procédé de décontamination de surfaces selon l'invention permet l'élimination de toutes sortes d'espèces contaminantes (aussi appelées contaminants ou polluants) et notamment des espèces contaminantes ioniques, chimiques, biologiques, ou nucléaires, radioactives.

La présente invention a trait en outre à un procédé de décontamination de milieux gazeux contaminé par des espèces contaminantes en suspension dans ces milieux gazeux.

Le milieu gazeux peut être de l'air et alors le procédé selon l'invention est qualifié de procédé de décontamination atmosphérique.

Les espèces contaminantes ou contaminants en suspension -qui peuvent notamment être les espèces contaminantes précitées- peuvent se présenter notamment sous la forme de particules solides, de particules liquides, ou encore sous la forme d'espèces moléculaires.

Plus exactement, le procédé de décontamination de milieux gazeux selon l'invention permet la capture, le piégeage, le rabattement et la fixation de ces contaminants en suspension, sur une surface solide et éventuellement la décontamination de cette surface.

Le domaine technique de l'invention, peut, de manière générale, être défini comme celui de la décontamination de surfaces de substrats, ou de milieux gazeux, par voie sèche en vue d'éliminer les polluants, contaminants qui se trouvent sur ces surfaces et/ou sous ces surfaces (en subsurface), c'est-à-dire incrustés dans les premières couches de ces substrats, ou en suspension dans ces milieux gazeux, et dont la présence sur et sous ces surfaces (en subsurface), c'est-à-dire incrustés dans les premières couches de ces substrats, ou la présence en suspension dans ces milieux gazeux, n'est pas souhaitée.

Autrement dit, le domaine technique de l'invention est le domaine de la décontamination ionique, biologique ou chimique, de la désinfection, du nettoyage, du dégraissage de surfaces et de la dépollution des solides et des atmosphères.

Les applications du procédé de décontamination de surfaces ou de milieux gazeux selon l'invention sont nombreuses et variées et concernent de nombreux domaines d'activité, industriels et domestiques.

Plus spécifiquement, les procédés selon l'invention peuvent être utilisés dans le domaine de l'assainissement, démantèlement des installations nucléaires et la maintenance des installations nucléaire en cas de contamination de surfaces.

### ÉTAT DE LA TECHNIQUE ANTÉRIEURE

FR 2 984 170 enseigne un gel ferromagnétique constitué par une solution colloïdale comprenant un agent viscosant inorganique, un composé ferromagnétique et un solvant.

Les gels « aspirables » de décontamination sont des suspensions colloïdales concentrées de particules minérales, par exemple de particules de silice ou d'alumine. Ces particules minérales jouent le rôle de gélifiant viscosant d'une solution liquide. La solution liquide du gel contient un ou plusieurs réactifs de décontamination et éventuellement des co-viscosants, comme des tensioactifs, permettant notamment d'ajuster les propriétés rhéologiques. Ces différents constituants sont choisis pour formuler un gel que l'on peut facilement déposer ou pulvériser à l'aide de matériels adaptés.

Les gels aspirables permettent de s'affranchir des problèmes liés au caractère pulvérulent du déchet sec, et d'accroître l'efficacité du procédé mettant en œuvre un gel.

Ces gels sont aujourd'hui développés pour deux applications, qui sont : (1) le traitement, plus exactement la décontamination de surfaces et (2) le traitement, plus exactement la décontamination de milieux gazeux, notamment la décontamination atmosphérique.

### (1) Traitement de surfaces à l'aide de gels aspirables.

Dans le cadre du traitement de surfaces, les gels sont déposés ou pulvérisés en couches minces sur la surface à traiter. Grâce à leurs propriétés rhéologiques, les gels adhèrent sur tous types de parois, même verticales, sans couler. La présence d'un réactif de décontamination dans la formulation des gels permet une action corrosive ou dissolvante des couches de surface, libérant la contamination incrustée. Les contaminants sont alors solubilisés et/ou absorbés au sein des gels. Finalement, les gels sèchent, se fracturent, et produisent des résidus solides non-pulvérulents, appelés « paillettes », contenant la contamination. Ces paillettes sont facilement éliminées par voie sèche, notamment par aspiration, d'où la dénomination de gels « aspirables ». Certains principes actifs, par exemple dans les gels dits gels « BC » permettent également de dégrader la contamination biologique et/ou chimique en la rendant inoffensive.

Les procédés de décontamination qui mettent en œuvre ces gels aspirables sont donc des procédés de décontamination de surface par voie sèche, ne générant aucun effluent liquide et peu de résidus solides secs. En effet, ces résidus solides secs ne représentent en moyenne qu'un quart de la masse de gel initialement pulvérisée. De plus, ces procédés limitent le temps d'exposition des opérateurs à la contamination radioactive, du fait de leur mise en œuvre facile par pulvérisation puis aspiration des résidus secs, et du fait que la présence de l'opérateur n'est pas requise pendant le séchage du gel.

Le traitement de surfaces à l'aide de gels aspirables a été mis en œuvre dans le cadre de différentes applications.

En effet, en adaptant la formulation des gels, on peut notamment les utiliser pour des opérations de décontamination nucléaire, de dépollution ou de dégraissage de surface [1-2], de décontamination biologique de surface [3] ou d'élimination de couches organiques déposées en surface d'un substrat solide [4].

Quel que soit le domaine d'application, les gels aspirables sont mis en œuvre selon un procédé en deux étapes :

### Première étape : Application du gel sur la surface à traiter.

Cette application peut être réalisée de différentes façons :
- Manuellement par talochage. Cette technique consiste à déposer du gel sur la surface avec une spatule ou taloche, puis à l'étaler à la manière d'un enduit. Cette technique est facilement réalisable sur des petites surfaces planes. Cependant elle devient difficile à mettre en œuvre lorsque la surface est grande ou de géométrie plus complexe. Par exemple, il est impossible, ou très difficile pour l'opérateur d'étaler la couche de gel à l'intérieur d'une tuyauterie ou canalisation. De plus, la présence de l'opérateur à proximité de la surface contaminée est nécessaire, ce qui peut s'avérer problématique dans le cadre d'opérations de décontamination nucléaire ou biologique notamment.
- Par pulvérisation. Cette technique consiste à déposer le gel avec un dispositif de pulvérisation, ceci facilite et accélère l'application de la couche de gel, notamment sur de grandes surfaces planes. Cependant, il est impossible pour l'opérateur d'appliquer le gel sur des surfaces difficilement accessibles au jet émis par le dispositif de pulvérisation, comme à l'intérieur d'une canalisation ou tuyauterie.

Plus généralement, aussi bien l'application par talochage que l'application par pulvérisation des gels sont des techniques satisfaisantes quand la surface est aisément accessible, par exemple sur un mur ou sur un sol. Cependant, pour des zones difficiles d'accès, comme à l'intérieur d'une canalisation, il devient assez complexe, voire impossible, d'appliquer les gels, et on préférera alors utiliser des solutions liquides ou des mousses. Mais ces solutions et mousses génèrent des effluents liquides contaminés qui doivent ensuite mis en conformité pour un traitement dans les Stations de Traitement des Effluents Liquides (STEL) ou un rejet dans l'environnement.

Deuxième étape : Récupération des résidus, déchets secs.

Après application des gels, action des gels sur la surface (décontamination, dégraissage...) et séchage, les gels aspirables forment des résidus, déchets secs. On peut alors récupérer ces résidus de la manière suivante :
- Soit manuellement à l'aide d'une brosse et d'une pelle pour ensuite les transférer dans un conteneur adapté. Cette opération manuelle est particulièrement pénible et répétitive pour les opérateurs et allonge leur temps d'exposition à la contamination, ce qui est particulièrement problématique dans le cas d'opérations de décontamination nucléaire ou biologique notamment.
- Soit à l'aide d'un aspirateur électrique qui a l'avantage d'éviter un contact proche entre l'opérateur et le résidu contenant la contamination, et de mettre directement les résidus dans un contenant adapté d'autre part. Cependant, les opérations d'aspiration peuvent engendrer la remise en suspension de particules. Des filtres THE doivent donc être utilisés en sortie de l'aspirateur et vont devenir des déchets secondaires supplémentaires. Par ailleurs, la récupération des résidus solides peut s'avérer complexe si ces derniers sont présents dans des zones inaccessibles pour le dispositif d'aspiration qui est souvent assez volumineux.

### (2) Traitement de milieux gazeux, atmosphères à l'aide de gels aspirables.

Le document [5] décrit un procédé de décontamination d'un milieu gazeux tel que de l'air contaminé par des espèces contaminantes en suspension dans lequel on pulvérise dans ce milieu gazeux de fines gouttelettes formant ainsi un brouillard d'un gel inorganique. Les espèces contaminantes sont alors captées, capturées par les gouttelettes de gel et les gouttelettes vont se déposer et adhérer sur les parois du volume entourant le milieu gazeux (mur, sols, plafonds). Les gouttelettes déposées vont ainsi former une couche de gel qui va finalement sécher et former un résidu sec contenant les espèces contaminantes en suspension qui ont été captées.

Ce procédé permet ainsi la capture et la fixation de contaminants en suspension dans un milieu gazeux, mais ce procédé présente plusieurs inconvénients :
- Certaines gouttelettes, parfois très fines, mettent du temps à retomber et peuvent rester jusqu'à plusieurs heures en suspension, engendrant alors des opérations de décontamination très longues.
- La décontamination de volumes étroits, tels que des conduits d'aération, peut s'avérer problématique car l'endroit où est formé le brouillard de gel est soumis aux contraintes inhérentes au dispositif de pulvérisation qui ne peut accéder à de tels volumes.
- Le dépôt des gouttelettes de gels doit parfois être évité sur certaines surfaces sensibles et la précision de la pulvérisation ne le permet pas.
- Il est parfois complexe de récupérer le déchet solide final contenant les contaminants, particulièrement si les gouttelettes se sont déposées dans des zones difficiles d'accès.
- La génération de très fines gouttelettes peut également engendrer la formation de déchets solides de petite taille qui peuvent se remettre en suspension, notamment lorsque des procédés de récupération par aspiration sont utilisés.

Il existe donc, au regard de ce qui précède, un besoin pour un procédé de décontamination d'une surface, et pour un procédé de décontamination d'un volume d'un milieu gazeux, mettant en œuvre un gel, notamment un gel aspirable (que ce gel soit appliqué par talochage ou par pulvérisation), qui ne présentent pas les défauts, inconvénients, et désavantages des procédés de l'art antérieur tels qu'ils ont été exposés dans ce qui précède.

Il existe encore un besoin pour un procédé de décontamination mettant en œuvre un gel, qui apporte une solution aux problèmes des procédés de l'art antérieur tels qu'ils ont été mentionnés plus haut.

### EXPOSÉ DE L'INVENTION

Ce but, et d'autres encore, sont atteints, conformément à l'invention, par un procédé de décontamination d'au moins une surface d'un substrat en un matériau solide, ladite surface étant contaminée par au moins une espèce contaminante se trouvant sur ladite surface et/ou sous ladite surface (en subsurface) dans les premières couches du substrat, dans lequel on réalise au moins un cycle comprenant les étapes successives suivantes :
a) on applique un gel ferromagnétique inorganique constitué par une solution colloïdale comprenant un agent viscosant inorganique, un composé ferromagnétique, et un solvant, sur ladite surface, puis on déplace et on étale à distance le gel appliqué sur la surface à l'aide d'un aimant ;
b) on maintient le gel sur la surface au moins pendant une durée suffisante pour que le gel détruise et/ou inactive et/ou dégrade et/ou absorbe l'espèce contaminante, et pour que le gel sèche et forme sur la surface un résidu sec et solide contenant ladite espèce contaminante ;
c) on déplace et on rassemble sur la surface, à l'aide d'un aimant, le résidu sec et solide contenant ladite espèce contaminante, et on récupère le résidu sec et solide contenant ladite espèce contaminante.

Le substrat est donc solide et la surface de ce substrat est également solide.

Avantageusement, le substrat en un matériau solide peut être en un matériau choisi parmi les métaux et les alliages métalliques comme l'acier inoxydable, les aciers peints, l'aluminium et le plomb ; les polymères tels que les matières plastiques ou les caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments et les matériaux cimentaires; les mortiers et bétons ; les plâtres ; les briques ; la pierre naturelle ou artificielle ; les céramiques.

L'espèce contaminante peut être choisie parmi les espèces contaminantes ioniques, chimiques, biologiques, nucléaires ou radioactives.

L'espèce contaminante peut notamment être choisie parmi toutes les espèces contaminantes énumérées plus bas lors de la description du gel, ainsi que lors de la description du procédé de décontamination d'un milieu gazeux.

Notamment, l'espèce contaminante peut être une espèce contaminante ionique choisie parmi les ions métalliques monovalents et multivalents, notamment parmi les ions métalliques monovalents et multivalents toxiques comme les ions chrome (VI), nickel (II), argent (I), cadmium (II) , mercure (II), arsenic (III), et plomb (II).

L'espèce contaminante peut être une espèce contaminante biologique, choisie parmi les bactéries, les champignons, les levures, les virus, les toxines, les spores notamment les spores de Bacillus anthracis, les prions, et les protozoaires.

En particulier, l'espèce contaminante biologique peut être choisie parmi les espèces biotoxiques telles que les spores pathogènes comme par exemple les spores de Bacillus anthracis, les toxines comme par exemple la toxine botulique ou la ricine, les bactéries comme les bactéries Yersinia pestis, les prions, et les virus comme les coronavirus, le virus de la vaccine ou les virus des fièvres hémorragiques par exemple de type Ebola.

L'espèce contaminante peut être choisie parmi les espèces chimiques toxiques, telles que les gaz toxiques, en particulier neurotoxiques ou vésicants, notamment les composés organophosphorés, comme le Sarin ou agent GB, le VX, le Tabun ou agent GA, le Soman, le Cyclosarin, le diisopropyl fluoro phosphonate (DFP), l'Amiton ou agent VG, le Parathion, le gaz moutarde ou agent H ou agent HD, la Lewisite ou agent L, l'agent T.

L'espèce contaminante peut être une espèce contaminante radioactive et/ou chimiquement toxique, et/ou toxique du fait de sa forme et/ou de sa taille.

L'espèce contaminante toxique du fait de sa forme et/ou de sa taille, peut être une espèce contaminante se présentant sous la forme de particules solides telles que des microparticules, ou des nanoparticules, par exemple sous la forme de fibres telles que des microfibres ou des nanofibres, sous la forme de nanotubes, ou sous la forme de cristaux tels que des nanocristaux.

Notamment, l'espèce contaminante peut être l'amiante.

Avantageusement, le gel ferromagnétique inorganique peut être appliqué sur la surface à décontaminer à raison de 100 g à 2000 g de gel par m² de surface, de préférence de 500 à 1500 g de gel par m² de surface, de préférence encore de 600 à 1000 g de gel par m² de surface, ce qui correspond généralement à une épaisseur de gel déposé sur la surface comprise entre 0,5 mm et 2 mm.

Avantageusement, lors de l'étape b), une importante épaisseur de gel, par exemple une épaisseur de 2 mm 2 cm, peut être maintenue sur la surface à l'aide d'un aimant.

Cela permet notamment d'augmenter la profondeur de corrosion et l'efficacité de la décontamination.

Avantageusement, lors de l'étape a), on applique le gel ferromagnétique inorganique sur ladite surface par pulvérisation, au pinceau ou avec une taloche, puis on déplace et on étale à distance le gel appliqué sur la surface à l'aide d'un aimant.

Avantageusement, la surface à décontaminer peut être une surface difficile, voire impossible, à atteindre par pulvérisation, au pinceau ou avec une taloche, par exemple la surface interne d'un tuyau ou d'une canalisation.

Le gel ferromagnétique inorganique est alors déposé sur la surface interne à l'entrée du tuyau ou de la canalisation et est ensuite déplacé et étalé sur la surface interne à l'aide d'un aimant placé au voisinage de la surface externe ou sur la surface externe du tuyau ou de la canalisation.

Avantageusement, le séchage est réalisé à une température de 1°C à 50°C, de préférence de 15°C à 25°C, et sous une humidité relative de 20% à 80%, de préférence de 20% à 70%.

Avantageusement, le gel est maintenu sur la surface pendant une durée de 2 à 72 heures, de préférence de 2 à 48 heures, de préférence encore de 4 à 24 heures.

Avantageusement, le résidu sec et solide se présente sous la forme de particules, par exemple de paillettes, d'une taille de 1 à 10 mm, de préférence de 2 à 5 mm.

Avantageusement, le résidu sec et solide contenant ladite espèce contaminante est récupéré à l'aide d'un aimant et/ou par brossage et/ou par aspiration, par exemple avec un aspirateur muni d'un aimant.

Avantageusement, le cycle est répété de 1 à 10 fois en utilisant le même gel lors de tous les cycles, ou en utilisant des gels différents lors d'un ou de plusieurs cycle(s).

L'invention concerne aussi un procédé de décontamination d'un volume d'un milieu gazeux contaminé par des espèces contaminantes en suspension, ledit volume d'un milieu gazeux étant en contact avec au moins une surface d'un substrat solide, ledit procédé comprenant les étapes successives suivantes :
a) on pulvérise dans ledit volume d'un milieu gazeux de fines gouttelettes, formant ainsi un brouillard, d'un gel ferromagnétique inorganique constitué par une solution colloïdale comprenant un agent viscosant inorganique, un composé ferromagnétique et un solvant;
b) les espèces contaminantes en suspension sont capturées, captées par lesdites gouttelettes du gel ferromagnétique ;
c) les gouttelettes du gel ferromagnétique contenant les espèces contaminantes en suspension capturées sont déplacées sous l'action d'un aimant jusqu'à ce qu'elles se déposent et s'accumulent sur une zone déterminée de ladite surface du substrat solide ;
d) on maintient le gel sur la zone déterminée de la surface du substrat solide au moins pendant une durée suffisante pour que le gel sèche et forme un résidu sec et solide contenant les espèces contaminantes en suspension capturées ;
e) on récupère le résidu sec et solide contenant lesdites espèces contaminantes en suspension capturées.

Pour certains aspects du procédé selon l'invention de décontamination d'un volume gazeux, notamment en ce qui concerne les étapes a) et b), on pourra se référer au document [5] ou à la demande internationale PCT correspondante.

Avantageusement, le milieu gazeux peut être de l'air.

Avantageusement, le volume d'un milieu gazeux peut être un volume clos défini par des parois, telles qu'un sol, un plafond et des murs, formant ladite surface, le brouillard de fines gouttelettes remplit la totalité du volume clos, et les fines gouttelettes du gel ferromagnétique contenant les espèces contaminantes en suspension capturées se déposent sur au moins l'une des parois, de préférence sur une paroi inférieure telle qu'un sol.

Avantageusement, les fines gouttelettes ont une taille, définie par leur plus grande dimension, telle qu'un diamètre, de 1 à 1000 µm, de préférence de 5 à 200 µm.

Les espèces contaminantes en suspension peuvent être choisies parmi toutes les espèces contaminantes déjà énumérées plus haut, lors de la description du procédé de décontamination d'une surface, et plus bas lors de la description du gel, à l'exception généralement toutefois des espèces ioniques.

Les espèces contaminantes en suspension peuvent se présenter sous la forme de particules solides, de particules liquides, ou sous la forme d'espèces moléculaires.

Les espèces contaminantes peuvent être choisies parmi les espèces contaminantes chimiques, biologiques, nucléaires ou radioactives.

Les espèces contaminantes en suspension peuvent être des espèces contaminantes radioactives, et/ou chimiquement toxiques, et/ou toxiques du fait de leur forme et/ou de leur taille.

Les espèces contaminantes en suspension toxiques du fait de leur forme et/ou de leur taille, peuvent être choisies parmi les espèces contaminantes se présentant sous la forme de particules solides telles que des microparticules, ou des nanoparticules, par exemple sous la forme de fibres telles que des microfibres ou des nanofibres, sous la forme de nanotubes, ou sous la forme de cristaux tels que des nanocristaux.

En particulier, les espèces contaminantes en suspension peuvent être choisies parmi les métaux et métalloïdes sous forme de métal, de métalloïde, ou ionique, de préférence parmi les métaux dits « métaux lourds », et les métaux et métalloïdes toxiques sous forme de métal, de métalloïde, ou ionique; les composés de ces métaux et métalloïdes tels que les composés organométalliques, les sels de métaux, les oxydes de métaux, les carbures de métaux, etc. ; les céramiques ; le bois ; les céréales ; la farine ; l'amiante ; et les verres, par exemple sous la forme de laine de verre.

Notamment, l'espèce contaminante en suspension peut être l'amiante.

Avantageusement, le résidu sec et solide contenant lesdites espèces contaminantes en suspension capturées peut être récupéré à l'aide d'un aimant et/ou par brossage et/ou par aspiration par exemple avec un aspirateur muni d'un aimant.

Avantageusement, l'aimant est un électroaimant.

En effet, l'utilisation d'un électroaimant lors de l'étape a) ou de l'étape c) du procédé de décontamination d'au moins une surface ou lors de l'étape e) du procédé de décontamination d'un volume d'un milieu gazeux c'est-à-dire lors de l'étalement du gel ou lors de la récupération des résidus secs solides facilite le procédé de décontamination car :
- L'électroaimant peut être amené éteint, inactivé jusqu'à la zone d'intérêt déterminée, puis activé uniquement pour déplacer le gel. Cela permet d'éviter des aimantations parasites qui pourraient être néfastes, en fonction des zones de mise en œuvre du gel.
- De la même manière, l'électroaimant peut être activé pour récupérer les déchets, résidus solides puis désactivé afin de libérer les déchets solides et les transvaser dans un conteneur adapté.

Avantageusement, l'aimant est déplacé par télémanipulation, télé-opération.

En effet, l'étalement et la récupération du gel à l'aide d'un aimant peuvent être réalisés par un opérateur mais peuvent être également réalisés par télémanipulation. En effet, dans le cas de zones difficilement accessibles car fortement contaminées, le dépôt du gel, son étalement puis la récupération des déchets solides sont beaucoup plus faciles à réaliser à l'aide d'un aimant par télémanipulation que la mise en œuvre de gels aspirables par pulvérisation puis aspiration.

Le procédé selon l'invention, aussi bien pour la décontamination de surfaces, que pour la décontamination de milieux gazeux met en œuvre, un gel spécifique, qui est un gel ferromagnétique inorganique constitué par une solution colloïdale comprenant un agent viscosant inorganique, un composé ferromagnétique, et un solvant.

Par « composé ferromagnétique », on entend un composé sensible à l'action d'un champ magnétique, plus exactement un composé sensible à l'action d'un champ magnétique généré par un aimant, notamment un composé pouvant être attiré par un aimant ou formant un aimant permanent.

Le gel mis en œuvre dans le procédé selon l'invention, du fait qu'il contient un composé ferromagnétique peut être dénommé « gel ferromagnétique ».

Le gel mis en œuvre dans le procédé selon l'invention peut être aussi dénommé « gel aimantable » car du fait qu'il contient un composé ferromagnétique, il est sensible à l'action d'un champ magnétique généré par un aimant et il peut être attiré, déplacé par un aimant.

Le terme « gel » est parfaitement clair pour l'homme du métier et il a une signification largement acceptée.

Toutefois, généralement, on peut considérer qu'un gel peut posséder une viscosité supérieure ou égale à 0,1 Pa.s.

Généralement, la quantité de solvant est au minimum de 20% en masse, par rapport à la masse du gel, car sinon on n'obtient généralement pas un gel mais une mixture très pâteuse et non liée qui ne peut pas être correctement déplacée par un aimant.

Le gel mis en œuvre dans le procédé selon l'invention est qualifié de gel inorganique, minéral en ce sens qu'il comprend un agent viscosant inorganique, minéral.

Généralement, le gel ne contient en tant qu'agent viscosant, que cet agent viscosant inorganique, minéral, et il ne contient pas d'agent viscosant organique.

Le gel mis en œuvre dans le procédé selon l'invention peut généralement être défini comme étant un gel dit « gel aspirable ».

Le terme « gel aspirable » est un terme couramment usité dans ce domaine de la technique, il a une signification largement acceptée que l'on rappelle plus bas.

Un gel aspirable est intrinsèquement différent d'un gel qui n'est pas aspirable.

La présence dans le gel de décontamination mis en œuvre dans le procédé selon l'invention, d'un composé ferromagnétique est l'une des caractéristiques fondamentales qui différencie le procédé selon l'invention des procédés de l'art antérieur.

Un procédé de décontamination mettant en œuvre un gel de décontamination, dans lequel se trouve incorporé un composé ferromagnétique n'est ni décrit ni suggéré dans les documents de l'art antérieur, tel que représenté notamment par les documents [1] à [5] cités plus haut.

Le gel mis en œuvre dans le procédé selon l'invention peut donc être défini comme étant un gel aspirable classique auquel est ajouté un composé ferromagnétique.

Le procédé selon l'invention, qui met en œuvre un gel de décontamination particulier, contenant un composé ferromagnétique, permet, notamment du fait de la présence de ce composé ferromagnétique dans le gel, de surmonter tous les inconvénients des procédés de décontamination de surfaces et de milieux gazeux mettant en œuvre des gels aspirables de l'art antérieur, et d'apporter une solution aux problèmes posés par ces procédés.

La présence d'un composé ferromagnétique dans le gel mis en œuvre dans le procédé selon l'invention permet notamment, au contraire des procédés de l'art antérieur, de déplacer facilement le gel sous l'action d'un champ magnétique, par exemple en attirant le gel ferromagnétique avec un aimant. Le fait de pouvoir ainsi déplacer facilement le gel ferromagnétique sous l'action d'un champ magnétique, par exemple d'un champ magnétique créé par un aimant, apporte de nombreux avantages -exposés dans la présente- dans le cadre des procédés de décontamination de surfaces et de milieux gazeux et permet de surmonter les problèmes que présentaient ces procédés.

En outre, les résidus solides engendrés par le séchage du gel peuvent également être déplacés et récupérés facilement sous l'action d'un champ magnétique, par exemple d'un champ magnétique créé par un aimant, ce qui apporte également de nombreux avantages -exposés dans la présente- dans le cadre des procédés de décontamination de surfaces et de milieux gazeux, et permet de surmonter les problèmes que présentaient ces procédés.

Les gels aspirables mis en œuvre dans le procédé selon l'invention possèdent toutes les propriétés avantageuses connues des gels aspirables, et ces propriétés avantageuses ne sont en rien affectées par l'addition à ces gels d'un composé ferromagnétique qui apporte quant à lui d'autres propriétés avantageuses.

En particulier, l'addition de ce composé ferromagnétique au gel ne modifie en rien les étapes de préparation du gel aspirable.

Les composés ferromagnétiques sont faciles d'utilisation et s'incorporent dans la formulation du gel mis en œuvre dans le procédé selon l'invention sous forme de particules pendant l'étape d'agitation lors de la préparation du gel selon l'invention.

Il est aussi important de noter que l'addition de composés ferromagnétiques ne modifie pas significativement le comportement physico-chimique du gel notamment en ce qui concerne la rhéologie et fracturation au cours du séchage.

En conséquence, on peut adapter facilement la formulation du gel à la texture du gel qui est souhaitée en fonction de l'application visée. La formulation du gel est adaptée aux milieux acides, basiques et solvants des gels de décontamination.

Le procédé selon l'invention présente un certain nombre d'avantages, liés à la présence de composés ferromagnétiques dans les gels de décontamination que le procédé selon l'invention met en œuvre. Ces avantages sont entre autres :
- Pour l'application du gel sur une surface contaminée :
   La possibilité de déplacer le gel en utilisant un aimant permanent ou un électro aimant. Cette opération peut être effectuée :
   - soit à distance en déplaçant l'aimant au-dessus du gel pour former une couche homogène,
   - soit à travers une paroi en plaçant l'aimant sur la face opposée de la surface à décontaminer, ce qui est extrêmement avantageux car cela peut permettre d'étaler le gel à l'intérieur d'une canalisation, par exemple.

De manière générale, la possibilité de déplacer le gel en utilisant un aimant permet de déposer une couche de gel sur des surfaces difficilement accessibles, voire non atteignables, notamment par pulvérisation.

L'utilisation d'un aimant peut également permettre de maintenir le gel sur une zone spécifique (par exemple un point chaud sur une surface) avec une épaisseur importante et d'éviter toute coulure. Cela peut ainsi permettre une décontamination plus en profondeur de la surface.
- Pour la décontamination d'atmosphères :
   La possibilité d'attirer avec un aimant de fines gouttelettes de gel pour capter des aérosols en suspension. Cela peut permettre d'accélérer l'opération de décontamination d'une atmosphère en provoquant la chute des plus fines gouttelettes, qui sinon peuvent rester quelques heures en suspension.

La possibilité de déplacer un nuage de gouttelettes de gel en les attirant avec un aimant. Cela peut être avantageux pour décontaminer l'intérieur d'un volume étroit, comme un conduit d'aération.
- Pour la récupération des déchets solides après séchage du gel :
   La récupération des résidus de gel avec un aspirateur équipé d'un aimant permet de récupérer le déchet solide en évitant de mettre en place des flux importants (diminution des besoins en extraction) ainsi que des filtres **THE.**

De cette manière, on évite également l'éventuelle remise en suspension de particules pulvérulentes à cause des flux en présence.

La récupération des résidus peut aussi se faire simplement, et sans aspiration, en utilisant un électroaimant pour regrouper les résidus sur la face aimantée, puis en désactivant l'électroaimant au-dessus d'un conteneur « poubelle », les résidus se détachent et tombent directement dans le conteneur.

La rapidité de l'opération de récupération des résidus en passant simplement près de la surface permet la réduction du temps d'exposition des opérateurs.

L'utilisation d'un aimant pour récupérer les déchets solides est plus facilement réalisable par télémanipulation ou télé-opération que l'utilisation d'un aspirateur.

Avantageusement, la solution colloïdale comprend, en outre, un ou plusieurs composant(s) choisi(s) parmi les composants suivants :
- un agent tensioactif ;
- un agent actif de décontamination ;
- un agent piégeur (« *getter* ») pour piéger les espèces contaminantes gazeuses, notamment les espèces contaminantes gazeuses toxiques ou explosives, telles que l'hydrogène;
- un polymère superabsorbant ;
- un agent extractant des espèces contaminantes.

De préférence, la solution colloïdale, comprend, de préférence est constituée par :
- 1% à 40% en masse, de préférence 5% à 30% en masse, de préférence encore 5% à 25% en masse, mieux 8% à 20% en masse, par rapport à la masse du gel, d'au moins un agent viscosant inorganique ;
- 0,1% à 40% en masse, de préférence 5 à 30 % en masse, par rapport à la masse du gel d'au moins un composé ferromagnétique ;
- éventuellement, 0,05% à 2% en masse par rapport à la masse du gel, d'au moins un agent tensioactif ;
- éventuellement 0,05 à 10 mol/L de gel, de préférence 0,1 à 5 mol/L de gel, de préférence encore 1 à 2 mol/L de gel, d'au moins un agent actif de décontamination ;
- éventuellement, 0,1% à 5% en masse, par rapport à la masse du gel, d'au moins un agent piégeur (« *getter* ») pour capter, piéger les espèces contaminantes gazeuses, notamment les espèces contaminantes gazeuses toxiques ou explosives, telles que l'hydrogène;
- éventuellement 0,05% à 5% en masse, de préférence 0,05% à 2% en masse, par rapport à la masse du gel, d'au moins un polymère superabsorbant ;
- éventuellement, 0,1% à 5% en masse, par rapport à la masse du gel, d'au moins un agent extractant des espèces contaminantes;
- et le reste de solvant, la quantité de solvant étant au minimum de 20% en masse, par rapport à la masse du gel.

La somme des pourcentages en masse de tous les composants, constituants du gel est bien évidemment de 100% en masse.

Par « reste de solvant », on entend que le solvant est toujours présent dans la solution colloïdale et que la quantité de solvant est une quantité telle que, lorsqu'elle est ajoutée aux quantités des composants de la solution colloïdale autres que le solvant (que ces composants soient des composants obligatoires ou éventuels cités plus haut, ou encore d'autres composants additionnels optionnels cités ou non cités), la quantité totale de tous les composants de la solution colloïdale est de 100% en masse.

Il est à noter qu'il n'est pas nécessaire que le gel contienne un ou plusieurs parmi les composants optionnels cités ci-dessus. En effet, un gel comprenant uniquement le solvant l'agent viscosant et le composé ferromagnétique peut être mis en œuvre avec succès, et permet d'obtenir les effets et avantages énumérés plus haut.

Autrement dit, de préférence, la solution colloïdale comprend, de préférence est constituée par :
- 1% à 40% en masse, de préférence 5% à 30% en masse, de préférence encore 5% à 25% en masse, mieux 8% à 20% en masse, par rapport à la masse du gel, d'au moins un agent viscosant inorganique ;
- 0,1% à 40% en masse, de préférence 5 à 30 % en masse, par rapport à la masse du gel d'au moins un composé ferromagnétique ;
   et un ou plusieurs composant(s) choisi(s) parmi les composants suivants, dans les proportions suivantes :
- éventuellement, 0,05 % à 2% en masse par rapport à la masse du gel, d'au moins un agent tensioactif ;
- éventuellement 0,05 à 10 mol/L de gel, de préférence 0,1 à 5 mol/L de gel, de préférence encore 1 à 2 mol/L de gel, d'au moins un agent actif de décontamination ;
- éventuellement, 0,1% à 5% en masse, par rapport à la masse du gel, d'au moins un agent piégeur (« *getter* ») pour capter, piéger les espèces contaminantes gazeuses, notamment les espèces contaminantes gazeuses toxiques ou explosives, telles que l'hydrogène;
- éventuellement 0,05% à 5% en masse, de préférence 0,05% à 2% en masse, par rapport à la masse du gel, d'au moins un polymère superabsorbant ;
- éventuellement, 0,1% à 5% en masse, par rapport à la masse du gel, d'au moins un agent extractant des espèces contaminantes; - et le reste de solvant, la quantité de solvant étant au minimum de 20% en masse, par rapport à la masse du gel.

Autrement dit, ou bien, de préférence, la solution colloïdale comprend, de préférence est constituée par :
- 1% à 40% en masse, de préférence 5% à 30% en masse, de préférence encore 5% à 25% en masse, mieux 8% à 20% en masse, par rapport à la masse du gel, d'au moins un agent viscosant inorganique ;
- 0,1% à 40% en masse, de préférence 5 à 30% en masse, par rapport à la masse du gel d'au moins un composé ferromagnétique ;
- et le reste de solvant, la quantité de solvant étant au minimum de 20% en masse, par rapport à la masse du gel.

La présence d'un agent tensioactif dans le gel mis en œuvre dans le procédé selon l'invention influence favorablement et notablement les propriétés rhéologiques du gel. Ce tensioactif favorise notamment la reprise de viscosité du gel suite à son application ou à sa pulvérisation, nébulisation, atomisation sous la forme de fines gouttelettes et évite les risques d'épandage ou de coulure lorsque le gel est appliqué ou se dépose sur des surfaces verticales et des plafonds.

La présence dans le gel d'au moins un agent actif de décontamination permet d'éliminer, détruire, inactiver, tuer, extraire les espèces contaminantes.

La présence dans le gel d'au moins un agent extractant des espèces contaminantes, permet de faciliter la capture et la fixation des espèces contaminantes.

La présence dans le gel d'au moins un polymère superabsorbant permet d'améliorer les performances de décontamination des gels sur les surfaces poreuses, particulièrement les matériaux cimentaires, lorsque les contaminants, polluants, ont pénétré dans la profondeur de la porosité.

Le gel mis en œuvre dans le procédé selon l'invention est une solution colloïdale, ce qui signifie que le gel selon l'invention contient des particules solides inorganiques, minérales, d'agent viscosant dont les particules élémentaires, primaires, ont une taille généralement de 2 nm à 20 µm, de préférence de 2 nm à 200 nm.

Du fait de la mise en œuvre d'un agent viscosant généralement exclusivement inorganique, sans agent viscosant organique, la teneur en matières organiques du gel mis en œuvre selon l'invention est généralement inférieure à 4% en masse, de préférence inférieure à 2% en masse, ce qui constitue un avantage des gels mis en œuvre selon l'invention.

Ces particules solides, minérales, inorganiques jouent le rôle de viscosant pour permettre à la solution, par exemple la solution aqueuse, de se gélifier.

Avantageusement, l'agent viscosant inorganique peut être choisi parmi les oxydes de métaux tels que les alumines, les oxydes de metalloïdes tels que les silices, les hydroxydes de métaux, les hydroxydes de metalloïdes, les oxyhydroxydes de métaux, les oxyhydroxydes de métalloïdes, les aluminosilicates, les argiles telles que la smectite, et leurs mélanges.

En particulier, l'agent viscosant inorganique peut être choisi parmi les alumines (Al₂O₃) et les silices (SiO₂).

L'agent viscosant inorganique peut ne comprendre qu'une seule silice ou alumine ou un mélange de celles-ci, à savoir un mélange de deux silices différentes ou plus (mélange SiO₂/SiO₂), un mélange de deux alumines, différentes ou plus (mélange Al₂O₃/Al₂O₃), ou encore un mélange d'une ou plusieurs silices avec une ou plusieurs alumines (mélange SiO₂/Al₂O₃).

Avantageusement, l'agent viscosant inorganique peut être choisi parmi les silices pyrogénées, les silices précipitées, les silices hydrophiles, les silices hydrophobes, les silices acides, les silices basiques comme la silice Tixosil^{®} 73, commercialisée par la société Rhodia, et leurs mélanges.

Parmi les silices acides, on peut notamment citer les silices pyrogénées ou fumées de silice "Cab-O-Sil"^{®} M5, H5 ou EH5, commercialisées par la société CABOT^{®}, et les silices pyrogénées commercialisées par la société EVONIK INDUSTRIES^{®} sous l'appellation AEROSIL^{®}.

Parmi ces silices pyrogénées, on préférera encore la silice AEROSIL^{®} 380 d'une surface spécifique de 380 m²/g qui offre les propriétés viscosantes maximales pour une charge minérale minimale.

La silice utilisée peut aussi être une silice dite précipitée obtenue par exemple par voie humide par mélange d'une solution de silicate de soude et d'un acide. Les silices précipitées préférées sont commercialisées par la société EVONIK INDUSTRIES^{®} sous le nom de SIPERNAT^{®} 22 LS et FK 310 ou encore par la société RHODIA^{®} sous le nom de TIXOSIL^{®} 331, cette dernière est une silice précipitée dont la surface spécifique moyenne est comprise entre 170 et 200 m²/g.

Avantageusement, l'agent viscosant inorganique est constitué par un mélange d'une silice précipitée et d'une silice pyrogènée.

L'alumine peut être choisie parmi les alumines calcinées, les alumines calcinées broyées, et leurs mélanges.

De manière avantageuse, l'agent viscosant inorganique peut être constitué par une ou plusieurs alumine(s) représentant généralement de 5% à 30% en masse par rapport à la masse totale du gel.

Dans ce cas, la ou les alumine(s) est (sont) de préférence à une concentration de 8% à 17% en masse par rapport à la masse totale du gel pour assurer un séchage du gel à température comprise entre 20°C et 50°C et à une humidité relative comprise entre 20% et 60% en moyenne en 30 minutes à 5 heures.

Avantageusement, la ou les alumine(s) peut (peuvent) être choisie(s) parmi les alumines pyrogénées, de préférence parmi les alumines pyrogénées à granulométrie fine.

A titre d'exemple, on peut citer le produit vendu par la société EVONIK INDUSTRIES^{®} sous la désignation commerciale « Aeroxide Alumine C » qui est de l'alumine fine pyrogènée.

La nature de l'agent viscosant minéral, inorganique, notamment mais non exclusivement, lorsqu'il est constitué d'une ou plusieurs alumine(s), influence de manière inattendue le séchage du gel mis en œuvre dans le procédé selon l'invention et la granulométrie du résidu obtenu.

C'est également le cas lorsque l'agent viscosant est constitué d'une ou plusieurs silices et plus généralement d'un ou plusieurs oxydes de métaux ou de métalloïdes décrits plus haut.

Cette propriété n'est pas exclusive à l'alumine mais est une propriété commune, générale des gels aspirables.

En effet, le gel sec se présente sous la forme de particules de taille contrôlée, plus précisément de paillettes solides millimétriques, dont la taille va généralement de 1 à 10 mm, de préférence de 2 à 5 mm grâce notamment aux compositions précitées, en particulier, mais pas seulement, lorsque l'agent viscosant est constitué par une ou plusieurs alumine(s).

Là encore, c'est également le cas lorsque l'agent viscosant est constitué d'une ou plusieurs silices et, plus généralement, d'un ou plusieurs oxydes de métaux ou de métalloïdes décrits plus haut.

Précisons que la taille des particules correspond généralement à leur plus grande dimension.

En d'autres termes, les particules solides minérales du gel mis en œuvre selon l'invention, par exemple de type silice ou alumine, outre leur rôle de viscosant, jouent également un rôle fondamental lors du séchage du gel car elles assurent la fracturation du gel pour aboutir à un déchet sec sous forme de paillettes.

Le gel mis en œuvre dans le procédé selon l'invention peut contenir un agent actif de décontamination.

Cet agent actif de décontamination peut être tout agent actif de décontamination permettant d'éliminer une espèce contaminante quelle que soit la nature de cette espèce contaminante : que cette espèce contaminante soit chimique, biologique ou encore nucléaire, radioactive -en d'autres termes, cet agent de décontamination peut être tout agent de décontamination « NRBC » (Nucléaire, Biologique, Radiologique, Chimique)-, ou que cette espèce contaminante soit organique ou minérale, liquide ou solide.

Le gel mis en œuvre dans le procédé selon l'invention peut ainsi contenir un agent actif de décontamination biologique ou chimique ou encore nucléaire, radioactive.

L'agent actif de décontamination peut aussi être un agent de dégraissage, décapage, un agent corrosif afin d'éliminer une espèce contaminante éventuelle se trouvant sur la surface d'un substrat, et/ou sous la surface (en subsurface), c'est à dire incrustée dans les premières couches du substrat (à partir de la surface, depuis la surface), dans la profondeur du substrat.

Certains agents actifs de décontamination peuvent jouer simultanément plusieurs fonctions de décontamination.

Par agent de décontamination biologique que l'on peut aussi qualifier d'agent biocide, on entend tout agent, qui lorsqu'il est mis en contact avec une espèce biologique et notamment une espèce biologique toxique est susceptible d'inactiver ou de détruire celle-ci.

Par espèce biologique, on entend tout type de micro-organisme tel que les bactéries, les champignons, les levures, les virus, les toxines, les spores notamment les spores de *Bacillus anthracis,* les prions, et les protozoaires.

Les espèces biologiques qui sont éliminées, détruites, inactivées, par le gel mis en œuvre dans le procédé selon l'invention sont essentiellement des espèces biotoxiques telles que les spores pathogènes comme par exemple les spores de *Bacillus anthracis,* les toxines comme par exemple la toxine botulique ou la ricine, les bactéries comme les bactéries *Yersinia pestis*, et les virus comme les coronavirus, le virus de la vaccine ou les virus des fièvres hémorragiques par exemple de type Ebola.

Par agent de décontamination chimique, on entend tout agent qui lorsqu'il est mis en contact avec une espèce chimique et notamment une espèce chimique toxique est susceptible de détruire ou d'inactiver celle-ci.

Les espèces chimiques qui sont éliminées par le gel mis en œuvre dans le procédé selon l'invention sont notamment les espèces chimiques toxiques telles que les gaz toxiques, en particulier neurotoxiques ou vésicants.

Ces gaz toxiques sont notamment des composés organophosphorés, parmi lesquels on peut citer le Sarin ou agent GB, le VX, le Tabun ou agent GA, le Soman, le Cyclosarin, le diisopropyl fluoro phosphonate (DFP), l'Amiton ou agent VG, le Parathion. D'autres gaz toxiques sont le gaz moutarde ou agent H ou agent HD, la Lewisite ou agent L, l'agent T.

L'agent actif de décontamination, par exemple l'agent actif de décontamination biologique ou chimique peut être choisi parmi les bases telles que l'hydroxyde de sodium, l'hydroxyde de potassium, et leurs mélanges ; les acides tels que l'acide nitrique, l'acide phosphorique, l'acide chlorhydrique, l'acide sulfurique, les hydrogénooxalates comme l'hydrogénooxalate de sodium, et leurs mélanges ; les agents oxydants tels que les peroxydes, les permanganates, les persulfates, l'ozone, les hypochlorites tels que l'hypochlorite de sodium, les sels de cérium IV, et leurs mélanges ; les sels d'ammonium quaternaires tels que les sels d'hexadécylpyridinium (cétylpyridinium), comme le chlorure d'hexadécylpyridinium (cétylpyridinium); les agents réducteurs; et leurs mélanges.

Par exemple, l'agent actif de décontamination peut être un agent désinfectant tel que l'eau de Javel, qui apporte au gel des propriétés de décontamination, dépollution biologique et/ou chimique.

Certains agents actifs de décontamination peuvent être classés parmi plusieurs des catégories définies plus haut.

Ainsi, l'acide nitrique est-il un acide, mais aussi un agent oxydant, corrosif.

L'agent actif de décontamination, tel qu'un agent biocide, est généralement utilisé à une concentration de 0,05 à 10 mol/L de gel, de préférence de 0,1 à 5 mol/L de gel, de préférence encore de 1 à 2 mol/L de gel, afin de garantir un pouvoir de décontamination, par exemple un pouvoir d'inhibition des espèces biologiques, notamment biotoxiques, compatible avec le temps de séchage du gel, et pour assurer par exemple un séchage du gel à une température comprise entre 20°C et 50°C et à une humidité relative comprise entre 20% et 60 % en moyenne en 30 minutes à 5 heures.

De manière à atteindre l'efficacité totale, y compris dans les conditions de température et d'humidité les plus défavorables vis-à-vis du temps de séchage, la formulation du gel supporte différentes concentrations en agent actif. On peut remarquer, en effet, que l'augmentation de la concentration en agent de décontamination plus particulièrement en agent de décontamination acide ou basique accroît l'efficacité du procédé.

L'agent actif de décontamination peut être un acide ou un mélange d'acides. Ces acides sont généralement choisis parmi les acides minéraux tels que l'acide chlorhydrique, l'acide nitrique, l'acide sulfurique et l'acide phosphorique.

Les acides, tels que l'acide nitrique, ont une action corrosive qui permet d'éliminer une couche de surface d'un substrat solide contenant des espèces contaminantes (voir exemple 5).

Autrement dit, les acides sont généralement utilisés pour leur pouvoir corrosif permettant de libérer la contamination incrustée dans les premières couches (à partir de la surface) d'un substrat contaminé, notamment d'un substrat métallique.

L'acide ou les acides est (sont) de préférence présent(s) à une concentration de 0,5 à 10 mol/L, de préférence encore de 1 à 10 mol/L, mieux de 3 à 6 mol/L pour assurer un séchage du gel généralement à une température comprise entre 20°C et 50°C et à une humidité relative comprise entre 20% et 60% en moyenne en 30 minutes à 5 heures.

Pour ce type de gel acide, l'agent viscosant inorganique est de préférence la silice ou un mélange de silices.

Ou bien, l'agent actif de décontamination par exemple l'agent actif de décontamination biologique peut être une base, de préférence une base minérale, choisie de préférence parmi la soude, la potasse, et leurs mélanges.

Dans le cas d'une telle formulation de gel basique, le gel mis en œuvre dans le procédé selon l'invention a, outre l'action de décontamination, une action de dégraissage ce qui permet d'éliminer aussi les espèces contaminantes éventuelles en surface du substrat.

Comme on l'a déjà mentionné plus haut, de manière à atteindre une efficacité totale, y compris dans les conditions climatiques les plus défavorables vis-à-vis du temps de séchage du gel, le gel mis en œuvre dans le procédé selon l'invention peut présenter une large gamme de concentration en agent(s) de décontamination basique(s).

En effet, l'augmentation de la concentration en agent de décontamination basique comme NaOH ou KOH, jouant généralement le rôle d'agent biocide, permet d'accroître considérablement les vitesses d'inhibition des espèces biologiques, comme cela a été démontré pour des spores de *Bacillus thuringiensis.*

La base est avantageusement présente à une concentration inférieure à 10 mol/L, de préférence entre 0,5 et 7 mol/L, de préférence encore entre 1 et 5 mol/L, mieux entre 3 et 6 mol/L, pour assurer un séchage du gel à une température comprise entre 20°C et 50°C et à une humidité relative comprise entre 20% et 60% en moyenne en 30 minutes à 5 heures.

Pour ce type de gel alcalin, basique, l'agent viscosant inorganique est de préférence une alumine ou un mélange d'alumines.

L'agent de décontamination en particulier lorsqu'il s'agit d'un agent de décontamination biologique est de préférence l'hydroxyde de sodium ou l'hydroxyde de potassium.

Au regard par exemple de la cinétique d'inhibition des spores et des durées de séchage des gels en fonction de la température, l'agent actif de décontamination, notamment lorsqu'il s'agit d'un agent biocide sera de préférence l'hydroxyde de sodium à une concentration comprise entre 1 et 5 mol/L.

Pour la décontamination biologique, les agents de décontamination préférés sont la combinaison d'une base minérale et d'un agent oxydant comme cela est décrit dans le document [3], à la description duquel on pourra se référer.

Le gel peut éventuellement aussi contenir un agent tensioactif ou un mélange d'agents tensioactifs.

Avantageusement, l'agent tensioactif peut être choisi parmi les agents tensioactifs possédant une ou plusieurs parmi les propriétés mouillantes, les propriétés émulsifiantes et les propriétés détergentes ; et leurs mélanges.

L'agent tensioactif peut être choisi dans le groupe constitué par les alcoxylates d'alcool, les alkyl-aryl sulphonates, les alkyl phénol éthoxylates, les copolymères blocs à base d'oxyde d'éthylène et/ou d'oxyde de propylène, les alcools éthoxylés, les phosphates d'éther, les acides éthoxylés (légers et lourds), les esters de glycérol, les imidazolines, les sels d'ammonium quaternaires (quats), les alcanolamides, les oxydes d'amines, et les mélanges de ceux-ci.

Les agents tensioactifs sont de préférence des copolymères blocs commercialisés par la société BASF^{®} sous la dénomination PLURONIC^{®}.

Les Pluronics^{®} sont des copolymères séquencés d'oxyde d'éthylène et d'oxyde de propylène.

Les Pluronics^{®} sont généralement préférés pour les gels de décontamination biologique.

D'autres tensioactifs sont préférés pour les gels de décontamination nécessitant une action corrosive tels que les gels acides. On pourra à cet égard se référer au document [1].

Ces agents tensioactifs influencent les propriétés rhéologiques du gel, notamment le caractère thixotropique du produit et son temps de reprise et évitent l'apparition de coulure.

Les agents tensioactifs permettent, par ailleurs, de maîtriser l'adhésion du déchet sec, et de contrôler la taille des paillettes de résidu sec pour garantir la non-pulvérulence du déchet.

Avantageusement, le composé ferromagnétique est choisi parmi les métaux ferromagnétiques, comme le fer, le cobalt et le nickel ; les alliages ferromagnétiques comme les alliages de Heusler, et les alliages formant les aimants permanents tels que les aimants permanents aux terres rares comme le néodyme ou le dysprosium ou au cobalt ; et les ferrites.

Le composé ferromagnétique se présente généralement sous la forme de particules, par exemple de particules sphériques ou sphéroïdales.

Ces particules ont généralement une taille (définie par leur plus grande dimension telle que leur diamètre) de 2 nm à 20 µm.

Avantageusement, l'agent piégeur (« *getter* ») est choisi parmi les solides de très grande surface spécifique qui contiennent des sites de surfaces actifs, tels que les carbones poreux, par exemple les charbons actifs; les particules de matériaux cages, telles que les particules de zéolithes et de MOFs ; et les particules d'oxydes minéraux pouvant réagir avec les espèces contaminantes à piéger et plus spécifiquement avec l'hydrogène, tels que les oxydes de manganèse (MnO₃, Mn₂O₃, Mn₂O₄) et les oxydes de métaux nobles, par exemple Ag₂O ou RuO₂.

Avantageusement, l'agent extractant des espèces contaminantes est choisi parmi les adsorbants inorganiques comme les zéolithes, les argiles, les phosphates comme les apatites, les titanates comme les titanates de sodium, et les ferrocyanures et ferricyanures.

Cet agent extractant éventuel tel qu'une zéolithe ou une argile peut être utilisé dans le cas où l'espèce contaminante est un radionucléide, mais cet agent extractant éventuel peut aussi être utilisé dans le cas d'espèces contaminantes autres que les radionucléides, comme par exemple des métaux, tels que des métaux toxiques ou métaux lourds.

Par « polymère super-absorbant » également dénommé « SAP », on entend généralement un polymère capable, à l'état sec, d'absorber spontanément au moins 10 fois, de préférence au moins 20 fois son poids de liquide aqueux, en particulier d'eau et notamment d'eau distillée.

De tel polymères super-absorbants sont notamment décrits dans le document WO-A1-2013/092633 [7] auquel on pourra se référer.

Le solvant du gel mis en œuvre dans le procédé selon l'invention est généralement choisi parmi l'eau, les solvants organiques tels que les terpènes et les alcools et leurs mélanges.

Un solvant préféré est l'eau, et dans ce cas, le solvant est donc constitué par de l'eau, comprend 100% d'eau.

Ou bien, le solvant du gel mis en œuvre dans le procédé selon l'invention peut comprendre, de préférence être constitué par un ou plusieurs solvants organiques tels que des terpènes notamment le d-limonène.

L'utilisation d'un solvant organique dans le gel mis en œuvre dans le procédé selon l'invention permettra l'élimination de couches organiques (par exemple de type peinture, enduit ou tâche de bitume) contenant des espèces contaminantes, d'une surface d'un substrat solide.

D'autres caractéristiques et avantages de l'invention apparaîtront mieux à la lecture de la description détaillée qui suit, cette description étant faite à titre illustratif et non limitatif, en liaison avec les dessins joints.

### BRÈVE DESCRIPTION DES DESSINS

[Fig. 1] est une vue schématique d'un exemple d'une surface d'un substrat solide -à savoir la surface de l'intérieur d'une tuyauterie- qui peut être traitée par le procédé de décontamination d'une surface d'un substrat solide selon l'invention, utilisant un gel ferromagnétique inorganique.
[Fig. 2A] et
[Fig. 2B] et
[Fig. 2C] et
[Fig. 2D] sont des vues schématiques en coupe qui montrent les étapes successives d'un procédé selon l'invention de décontamination d'une surface d'un substrat solide utilisant un gel ferromagnétique inorganique.
[Fig. 3A] et
[Fig. 3B] sont des vues schématiques en coupe qui montrent les étapes successives d'un procédé selon l'invention de décontamination d'un volume d'un milieu gazeux contaminé par des espèces contaminantes en suspension utilisant un gel ferromagnétique inorganique.
[Fig. 3C] et
[Fig. 3D] sont des vues schématiques qui illustrent la récupération à l'aide d'un électroaimant des déchets secs et solides obtenus après une opération de décontamination réalisée conformément au procédé selon l'invention, avec un gel ferromagnétique.
[Fig. 4] est un graphique qui montre l'évolution de la viscosité (en Pa.s) en fonction du taux de cisaillement (en 1/s), pour les gels dits « *Gels 4, 9, 10 et 12 »* préparés dans l'exemple 1 (voir exemple 2).
[Fig. 5] est un graphique qui montre l'évolution de la contrainte de cisaillement (en Pa) en fonction de la déformation (en %) pour les gels dits « *Gels 4, 9, 10 et 12* » préparés dans l'exemple 1 (voir exemple 2).
[Fig. 6A] et
[Fig. 6B] et
[Fig. 6C] et
[Fig. 6D] sont des photographies qui montrent respectivement l'étalement avec un aimant des gels dits « *Gels 1, 2, 3, et 4 »* préparés dans l'exemple 1 (voir exemple 3).
[Fig. 7A] et
[Fig. 7B] et
[Fig. 7C] sont des photographies qui montrent le gel aimantable dit « *Gel13* » préparé dans l'exemple 1, déposé avant séchage (Figure 7A), après séchage (Figure 7B), et la récupération des résidus de gel sec après séchage à l'aide d'un aimant (Figure 7C) (voir exemple 4).
[Fig. 8A] et
[Fig. 8B] et
[Fig. 8C] et
[Fig. 8D] sont des photographies qui montrent la corrosion d'une surface d'une plaque d'acier par le gel aimantable dit « *Gel 13* » préparé dans l'exemple 1. La Figure 8A montre la plaque initiale sans gel, la Figure 8B montre la plaque avec le gel déposé, la Figure 8C montre la plaque avec le gel déposé après séchage, et la Figure 8D montre la plaque après élimination des résidus solide de gel sec (voir exemple 5).

### EXPOSÉ DÉTAILLÉ DE MODES DE RÉALISATION PARTICULIERS

Le gel mis en œuvre dans le procédé de décontamination selon l'invention (aussi bien d'une surface que d'un volume d'un milieu gazeux) peut être facilement préparé à la température ambiante.

Par exemple, le gel mis en œuvre dans le procédé selon l'invention peut être préparé en ajoutant de préférence progressivement, le composé ferromagnétique, au solvant tel que de l'eau, de préférence de l'eau déionisée, ou à un mélange du solvant et d'un ou plusieurs composants choisi(s) parmi les composants déjà énumérés plus haut, à savoir : un agent tensioactif; un polymère superabsorbant, un agent actif de décontamination; un agent piégeur (« *getter ») ;* un agent extractant des espèces contaminantes.

Ce mélange peut être réalisé par agitation mécanique, par exemple au moyen d'un agitateur mécanique équipé d'une hélice à trois pales. La vitesse de rotation est par exemple de 200 tours/minute, et la durée de l'agitation est par exemple de 3 à 5 minutes.

L'addition du composé ferromagnétique au solvant ou au mélange du solvant et du ou des composant(s) cité(s) plus haut peut être réalisée en versant simplement le composé ferromagnétique dans ledit mélange. Lors de l'addition du composé ferromagnétique, le mélange contenant le solvant, ce composé ferromagnétique, et éventuellement le ou composant(s) cité(s) plus haut est généralement maintenu sous agitation mécanique.

Cette agitation peut être, par exemple, réalisée au moyen d'un agitateur mécanique équipé d'une hélice à trois pales. L'agitation est poursuivie jusqu'à l'obtention d'une suspension homogène.

On ajoute ensuite, de préférence progressivement, le ou les agent(s) viscosant(s) inorganique(s) à cette suspension homogène toujours sous agitation.

La vitesse d'agitation est généralement augmentée graduellement au fur et à mesure que la viscosité de la solution augmente, pour atteindre finalement une vitesse d'agitation comprise par exemple entre 400 et 600 tours/minute lorsque la totalité du ou des agent(s) viscosant(s) inorganique(s) a été ajoutée, sans qu'il n'y ait eu de projections.

Après la fin de l'ajout du ou des viscosant(s) inorganique(s) (minéral (aux)), l'agitation est encore maintenue, par exemple pendant 2 à 5 minutes, de manière à obtenir un gel parfaitement homogène.

On laisse ensuite le gel ainsi préparé au repos pendant au moins une heure avant de l'utiliser.

Il est bien évident que d'autres protocoles de préparation des gels mis en œuvre dans le procédé selon l'invention peuvent être mis en œuvre avec une addition des composants du gel dans un ordre différent de celui mentionné plus haut.

Généralement, le gel mis en œuvre dans le procédé selon l'invention doit présenter une viscosité inférieure à 200 mPa.s sous un cisaillement de 1000s⁻¹ afin qu'il puisse être déposé ou pulvérisé, nébulisé, atomisé, sous la forme de fines gouttelettes telles que définies plus haut.

Le temps de reprise de la viscosité doit généralement être inférieur ou égal à une seconde et la viscosité sous faible cisaillement supérieure à 10 Pa.s pour ne pas couler sur une paroi lorsqu'il déposé ou pulvérisé avec une épaisseur inférieure à 1 mm.

Le gel ferromagnétique ainsi préparé peut être utilisé dans un procédé de décontamination d'une surface ou dans un procédé de décontamination d'un volume d'un milieu gazeux.

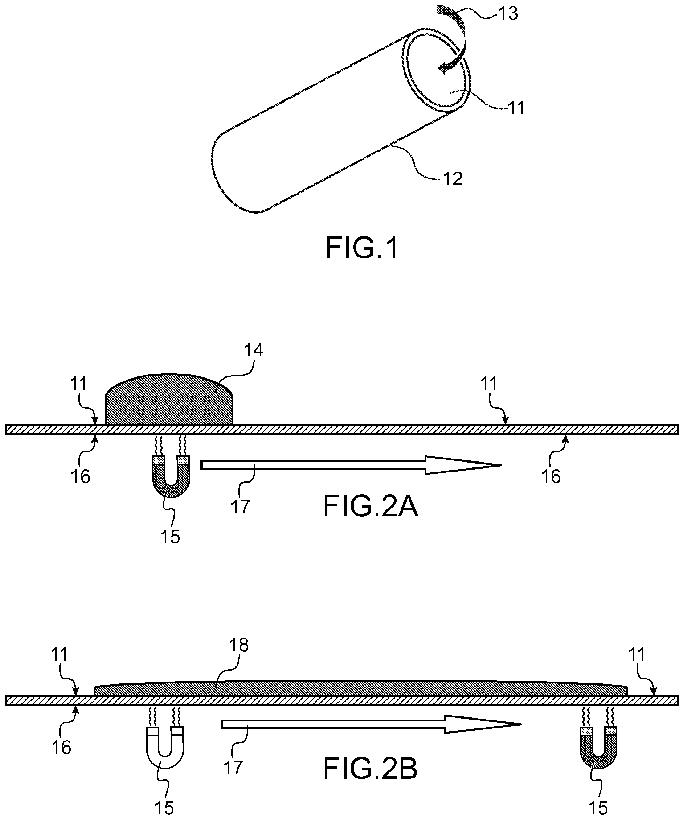

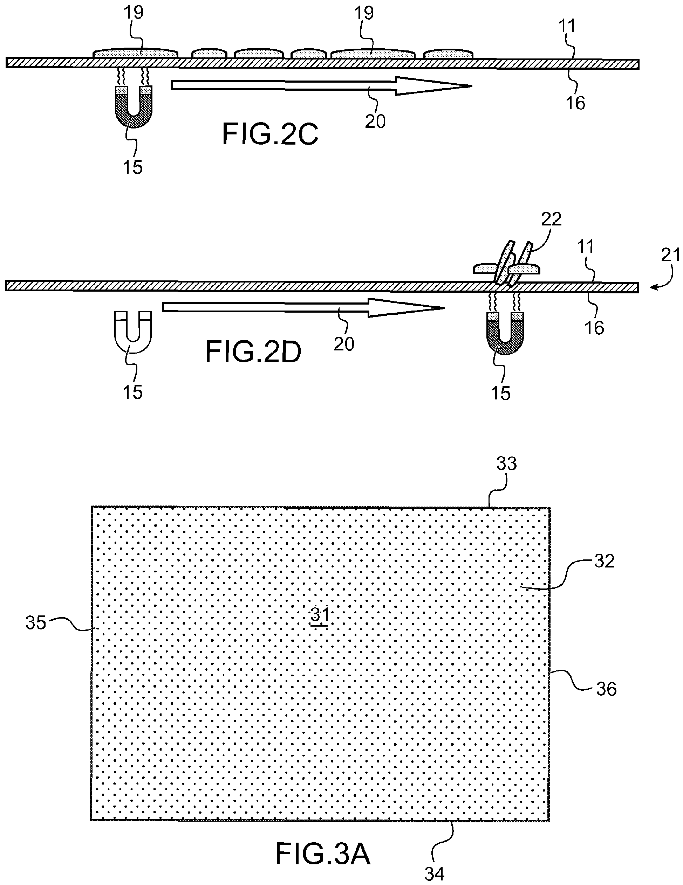

Les Figures 1, 2A, 2B, 2C, et 2D illustrent un procédé de décontamination de la surface interne (11) d'une canalisation, tuyau (12), à l'aide d'un gel ferromagnétique qui contient des particules ferromagnétiques. Cette surface n'est pas accessible à un gel qui serait appliqué par pulvérisation ou talochage.

Dans un premier temps (Figures 1 et 2A), on dépose une quantité suffisante de gel (14) ferromagnétique à l'entrée (13) de la canalisation sur la surface interne (11) à traiter de la canalisation (12).

Puis, on place un aimant (15) près de la surface externe (16) de la canalisation (12).

On déplace ensuite l'aimant (la flèche 17 montre le mouvement de l'aimant) (Figure 2B) le long de la surface externe (16), ce qui provoque un étalement du gel (14) à distance sur la surface interne (11) de la canalisation (12). On forme ainsi une couche millimétrique (18) de gel sur la surface interne (11) de la canalisation (12). On laisse cette couche (18) de gel sur la surface interne (11) de la canalisation (12) pendant la durée nécessaire pour réaliser la décontamination de cette surface interne (11).

On procède ensuite au séchage de la couche de gel (18), moyennant quoi des résidus solides de gel sec (19) sont formés sur la surface interne (11) de la canalisation (12) (Figure 2C).

Après séchage du gel et décontamination de la surface interne (11) de la canalisation (12), on peut alors utiliser de nouveau l'aimant (15) pour récupérer les résidus solides de gel sec (19) qui se trouvent sur la surface interne (11) de la canalisation (12).

Pour cela, on déplace l'aimant (la flèche 20 montre le mouvement de l'aimant) (Figures 2C et 2D) le long de la surface externe (16) de la canalisation. Les résidus solides de gel sec (19) qui contiennent toujours les particules ferromagnétiques, sont alors attirés par l'aimant (15) et déplacés vers une sortie (21) de la canalisation (12) où ils sont regroupés (22) et où ils peuvent être récupérés (Figure 2D).

A l'issue de l'étape illustrée sur la Figure 2D, la surface interne est propre et décontaminée.

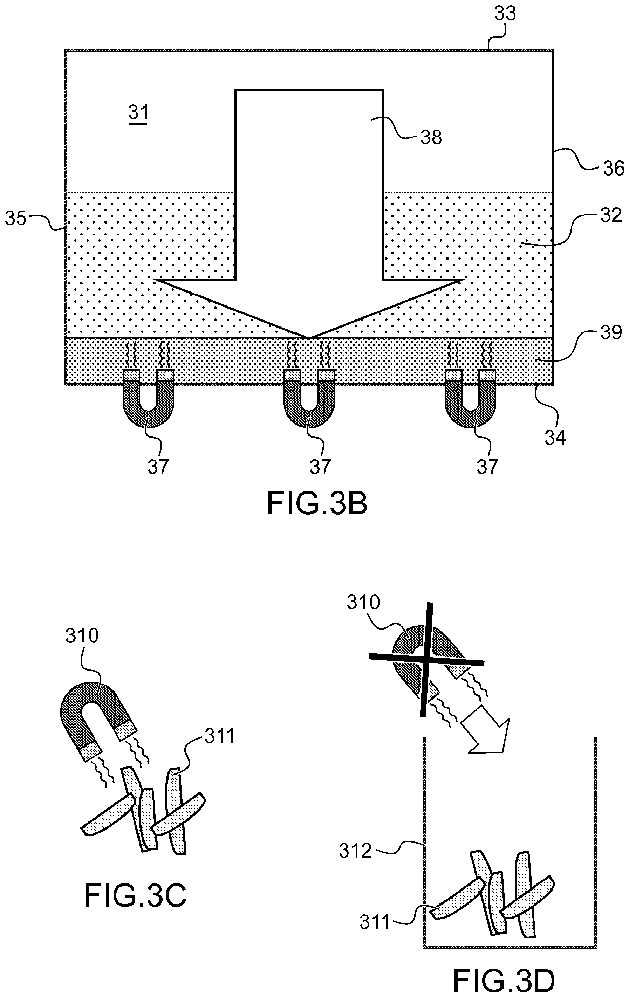

Les Figures 3A et 3B illustrent un procédé de décontamination d'un volume d'un milieu gazeux ou atmosphère à l'aide d'un gel ferromagnétique qui contient des particules ferromagnétiques.

Le gel ferromagnétique est pulvérisé sous la forme d'un brouillard de gouttelettes pour capter des aérosols d'espèces contaminantes en suspension dans un volume d'un milieu gazeux tel que de l'air.

La Figure 3A montre le volume (31) contenant les gouttelettes (32) de gel ferromagnétique qui y ont été pulvérisées, ce volume est défini par une paroi supérieure (33), une paroi inférieure (34) et des parois latérales (35 et 36) (les deux autres parois latérales ne sont pas montrées sur les Figures 3A et 3B).

Les gouttelettes (32) de gel vont capter les aérosols d'espèces contaminantes et les rabattre sur les parois (33, 34, 35, 36) du volume (31)

Afin d'accélérer l'opération de décontamination ou bien afin de déplacer les gouttelettes (32) de gel dans le volume (31) à décontaminer, un ou plusieurs aimants (37) sont utilisés. Sur la Figure 3B, trois aimants (37) sont ainsi mis en œuvre. Les gouttelettes de gel en suspension qui ont capté les espèces contaminantes vont alors être attirées par les aimants et se diriger dans la zone où se trouvent ces aimants.

De cette manière, on peut, d'une part, accélérer la chute de certaines gouttelettes très fines pouvant rester quelques heures en suspension et, d'autre part, définir la zone dans laquelle les gouttelettes vont pouvoir s'agglomérer et sécher pour finalement former un résidu sec et solide contenant les aérosols d'espèces contaminantes initialement présents dans le volume.

Sur la Figure 3B, les aimants (37) sont placés au voisinage de la paroi inférieure (34) et les gouttelettes en suspension sont attirées par les aimants en direction de la paroi inférieure (34). La flèche (38) montre le mouvement des gouttelettes en suspension attirées par les aimants. Les gouttelettes sont ainsi rabattues vers le bas plus rapidement, et vont s'agglomérer et sécher sur la paroi inférieure (34) pour former un résidu sec et solide (39) sur cette paroi.

Les Figures 3C et 3D montrent la récupération à l'aide d'un électroaimant des déchets secs et solides obtenus après une opération de décontamination réalisée conformément au procédé selon l'invention, avec un gel ferromagnétique.

L'utilisation d'un électroaimant permet une récupération aisée des déchets secs et solides contenant la contamination et le composé ferromagnétique.

Tout d'abord, l'électroaimant (310) est activé pour attirer les déchets, résidus secs et solides (311) sur la surface et les récupérer (Figure 3C).

L'électroaimant peut ensuite être déplacé sans qu'il n'y ait de risques de dissémination de la contamination car celle-ci est contenue dans les résidus solides aimantés.

Une fois placé au-dessus d'un conteneur (312) adapté, l'électroaimant (310) peut être désactivé (la désactivation est montrée par une croix noire) et les résidus solides (311) tombent dans le conteneur (312) qui peut être finalement dédié aux déchets.

L'invention va maintenant être décrite en référence aux exemples suivants, donnés à titre illustratif et non limitatif.

### EXEMPLES.

Exemple 1 : Préparation de gels ferromagnétiques mis en œuvre dans le procédé selon l'invention, et de gels comparatifs.

Dans cet exemple, on décrit la préparation de gels ferromagnétiques mis en œuvre dans le procédé selon l'invention, et de gels comparatifs, utilisés dans les exemples 2 à 6.

Les gels ferromagnétiques mis en œuvre dans le procédé selon l'invention 3, 4, 9, 10, 12, et 13 sont constitués par un liquide, un agent gélifiant, viscosant et des particules ferromagnétiques et les gels comparatifs 1 et 2, sont constitués seulement par un liquide et des particules ferromagnétiques. L'exemple 3 montre que les gels comparatifs 1 et 2, qui ne comprennent pas un agent viscosant, tel que l'alumine ou la silice ne rentrent pas dans le cadre des gels ferromagnétiques mis en œuvre dans le procédé selon l'invention.

Ces gels sont préparés selon le protocole suivant :
Le liquide, à savoir de l'eau désionisée ou de l'acide nitrique à 40%, est d'abord pesé dans un récipient adapté. Un composé ferromagnétique, à savoir de la ferrite, est ajouté progressivement dans le liquide sous agitation afin d'obtenir une suspension homogène.

L'agent viscosant, à savoir de l'alumine ou de la silice, est à son tour progressivement ajouté, toujours sous agitation. Après incorporation de la totalité des composants, un gel est obtenu puis laissé sous agitation pendant quelques minutes pour homogénéiser au mieux le mélange.

La ferrite est la ferrite « *Iron (II, III) oxide powder* », Fe₃O₄, commercialisée par SIGMA ALDRICH^{®}.

L'alumine est l'alumine Aeroxide^{®} Alu C commercialisée par EVONIK INDUSTRIES AG^{®} qui est une alumine pyrogènée d'une surface spécifique de 100 m²/g (BET).

La silice est la silice Aerosil^{®}380, commercialisée par EVONIK INDUSTRIES AG^{®} qui est une alumine pyrogénée d'une surface spécifique de 100 m²/g (BET).
Les formulations des gels ainsi préparés sont données dans le Tableau 1, ci-dessous :

**Tableau 1 : Formulation des gels utilisés dans les exemples 2 à 6.**

| Numéro du gel | Pourcentage massique de liquide | Pourcentage massique en alumine | Pourcentage massique en silice | Pourcentage massique en ferrite | Pourcentage massique total en phase solide |
|---|---|---|---|---|---|
| 1 | 50 (eau) | 0 | 0 | 50 | 50 |
| 2 | 66,7 (eau) | 0 | 0 | 33,3 | 33,3 |
| 3 | 47,7 (eau) | 23,8 | 0 | 28,5 | 52,3 |
| 4 | 41,5 (eau) | 37,7 | 0 | 20,8 | 58,5 |
| 9 | 63,3 (eau) | 20,9 | 0 | 15,8 | 36,7 |
| 10 | 60,2 (eau) | 24,7 | 0 | 15,1 | 39,8 |
| 12 | 83,2 (eau) | 0 | 6,9 | 9,9 | 16,8 |
| 13 | 76,2 (Acide nitrique 40%) | 0 | 9,5 | 14,3 | 23,8 |

Exemple 2 : Propriétés rhéologiques des gels ferromagnétiques mis en œuvre dans le procédé selon l'invention.

Dans cet exemple, on démontre que les gels ferromagnétiques mis en œuvre dans le procédé selon l'invention possèdent les propriétés rhéologiques nécessaires :
- pour être étalés sur une surface à distance à l'aide d'un aimant,
- pour tenir sans couler sur des surfaces verticales sur lesquels ils ont été déposés avec une épaisseur millimétrique.
Pour cela, il faut que les gels soient :
- Rhéofluidifiants : leur viscosité doit diminuer sous l'effet d'un cisaillement afin que les gels puissent s'écouler.
- Des fluides à seuil : Au repos, les gels ne doivent pas s'écouler afin de pouvoir tenir sur des surfaces verticales. Ils doivent donc posséder une contrainte seuil de quelques Pa. La contrainte seuil est la contrainte au-dessous de laquelle un gel ne s'écoule pas (il se comporte alors comme un solide) et au-dessus de laquelle il s'écoule (il se comporte alors comme un liquide).

Il est à noter que ces propriétés rhéologiques permettent également une application des gels par pulvérisation.

Les différentes mesures rhéologiques ont été réalisées à l'aide d'un rhéomètre TA Instruments DHR1 en géométrie Plan/Plan rugueux.

### Mise en évidence du caractère rhéofluidifiant des gels ferromagnétiques mis en œuvre dans le procédé selon l'invention.

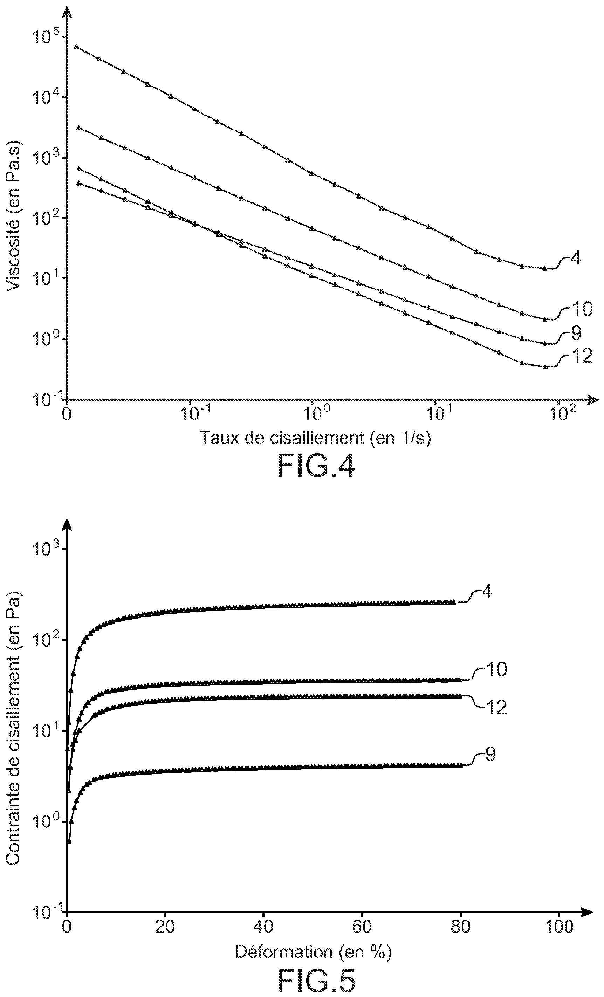

Dans un premier temps, la viscosité des gels a été mesurée en fonction du taux de cisaillement. Après un pré-cisaillement de 1 minute à un taux de cisaillement de 20 s⁻¹ puis 1 minute de repos, on applique une rampe continue de taux de cisaillement allant de 0,01 s⁻¹ à 100 s⁻¹ sur une durée totale de 60 secondes. La Figure 4 donne l'évolution de la viscosité (en Pa.s) des gels ferromagnétiques mis en œuvre dans le procédé selon l'invention en fonction du taux de cisaillement (en s⁻¹) pour des taux de cisaillement compris entre 0,01 et 100 s⁻¹.

Pour chacun des gels, on observe sur la Figure 4 une chute nette de la viscosité avec le taux de cisaillement, ce qui est caractéristique du comportement d'un fluide rhéofluidifiant.

Plus particulièrement, on observe que, pour les gels 4, 9 et 10, qui sont des gels ferromagnétiques mis en œuvre dans le procédé selon l'invention, comprenant de l'alumine comme agent viscosant, la viscosité des gels augmente avec le pourcentage massique total en solide.

Pour le gel 12 comprenant de la silice en tant qu'agent viscosant, on observe une chute un peu moins importante de la viscosité avec le taux de cisaillement (la pente de la droite est plus faible).

Tous les gels ferromagnétiques mis en œuvre dans le procédé selon l'invention présentent donc une viscosité importante à faible cisaillement (donc au repos) et une très faible viscosité lorsqu'ils sont cisaillés, ce qui leur permet d'être étalés sous l'effet d'un champ magnétique, ou pulvérisés.

### Mise en évidence de la contrainte seuil des gels de l'exemple 1.

Un faible taux de cisaillement (0,00673 s⁻¹) est appliqué aux gels de manière constante afin de les déformer en partant du repos, et ainsi de déterminer leur seuil d'écoulement. La Figure 5 représente l'évolution de la contrainte de cisaillement (en Pa) des gels en fonction de la déformation imposée (en %), à cisaillement constant, faible.

Pour chacun des gels, on observe que, tout d'abord, la contrainte augmente fortement en fonction de la déformation, le matériau est alors en régime solide (déformation élastique). On observe ensuite un changement de comportement : la contrainte atteint le seuil d'écoulement et le matériau passe en régime liquide (écoulement stationnaire). La contrainte seuil correspond alors à la contrainte au seuil d'écoulement des gels, les valeurs des contraintes seuil obtenues sont données dans le Tableau 2.

**Tableau 2 : Valeur des contraintes seuil des gels 4, 9, 10 et 12 préparés dans l'exemple 1.**

| Contrainte seuil gel 4 (en Pa) | Contrainte seuil gel 9 (en Pa) | Contrainte seuil gel 10 (en Pa) | Contrainte seuil gel 12 (en Pa) |
|---|---|---|---|
| 190 | 3 | 30 | 21 |

Le gel 4 possède ainsi une contrainte seuil importante car son pourcentage massique en phase solide est assez important. Lorsque l'on diminue la quantité de phase solide dans la formulation du gel, la contrainte seuil diminue (gel 10 puis gel 9). Comme précédemment, on observe un changement de comportement lorsque le viscosant est modifié. Ainsi, le gel 12 (avec de la silice comme agent viscosant) nécessite un pourcentage massique en phase solide moins important pour présenter une contrainte seuil significative de 21 Pa.

Dans le cadre d'opérations de décontamination de surface, des valeurs de contrainte seuil supérieures à 10 permettent aux gels, une fois étalés sur des surfaces à l'aide d'un aimant, de ne pas couler et d'y adhérer sur une épaisseur de quelques millimètres. Ainsi, parmi les gels de cet exemple, les gels 4, 10 et 12 peuvent être utilisés comme gels aimantables.

Dans le cadre d'opération de décontamination atmosphérique, de telles valeurs de contraintes seuils permettent aux gels, une fois déposés sur des surfaces après capture des contaminants, de ne pas couler et d'adhérer à ces surfaces sur une épaisseur de quelques millimètres.

En conclusion de cet exemple, il apparaît que les gels aimantables 4, 10 et 12 mis en œuvre dans le procédé selon l'invention possèdent bien les propriétés rhéologiques nécessaires pour une application à distance à l'aide d'un aimant dans le cadre d'opérations de décontamination de surface, mais aussi pour une pulvérisation sous la forme de fines gouttelettes pour des opérations de décontamination atmosphérique.

### Exemple 3 : Etalement des gels de l'exemple 1 à l'aide d'un aimant

Dans cet exemple, on démontre que les propriétés d'étalement des gels aimantables de l'exemple 1 dépendent de la présence dans les gels de particules ferromagnétiques mais aussi d'agents viscosants.

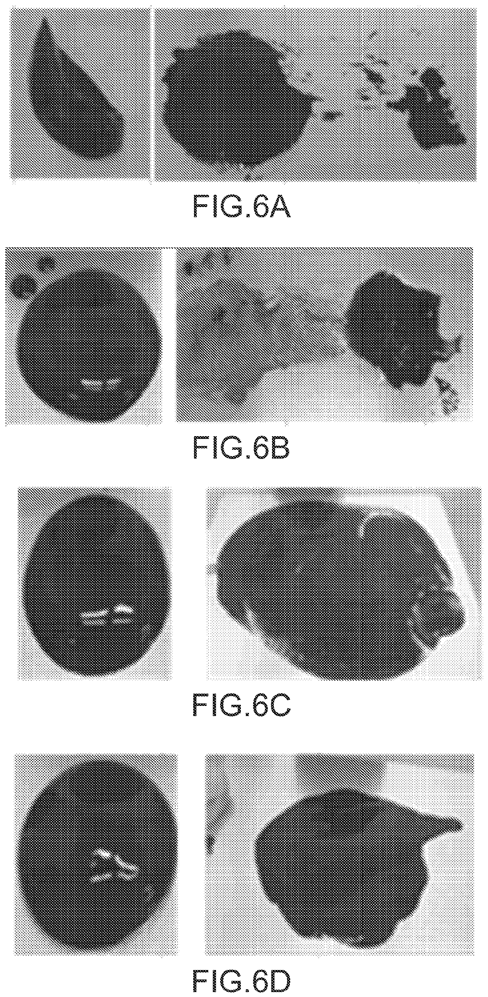

Pour cela, les gels 1, 2, 3 et 4 sont tout d'abord déposés sur une plaque d'aluminium. On utilise alors un aimant cylindrique de type N829 de chez ECLIPSE MAGNETICS (aimant au néodyme) que l'on déplace manuellement sur la face opposée de la plaque d'aluminium afin de tenter d'étaler le gel. Les Figures 6A, 6B, 6C et 6D, qui concernent respectivement les gels 1, 2, 3, et 4, présentent la tâche de gel initiale (à gauche) ainsi que la forme de cette dernière après le passage de l'aimant (à droite).
- Pour les gels comparatifs 1 et 2, qui ne comportent pas de viscosant (ni alumine, ni silice) : on observe que les gels, soit se déplacent partiellement en se fracturant (gel 1), soit « glissent » et n'adhèrent pas sur la surface (gel 2). Il n'y a donc pas d'étalement homogène, ce qui s'explique par l'absence de viscosant pour lier la formulation. Cet exemple montre bien que les gels comparatifs 1 et 2 ne peuvent pas être mis en œuvre dans le cadre du procédé de l'invention. En effet, même si ces gels comparatifs peuvent être déplacés par aimantation, ils ne permettent pas de déposer une couche de gel millimétrique. Pour cela, la présence d'un agent viscosant, tel que l'alumine ou la silice, est nécessaire.
- Pour les gels ferromagnétiques 3 et 4 mis en œuvre dans le procédé selon l'invention qui comportent un viscosant (alumine) : on observe un bon étalement du gel suivant le déplacement de l'aimant et une certaine épaisseur millimétrique de gel adhère sur le support alors qu'on déplace le restant sur la surface. La présence du viscosant permet de lier la formulation et donc d'avoir un étalement satisfaisant. On observe, par ailleurs, que le gel 4, qui possède un pourcentage massique en phase solide supérieur à celui du gel 3, s'étale moins bien. En effet, un même aimant étant utilisé, un gel plus visqueux est plus difficile à étaler. Il existe donc un lien entre propriétés rhéologiques du gel, force de l'aimant, et facilité d'étalement.

Exemple 4 : Fracturation du gel au séchage et récupération des déchets secs et solides.

Les gels aimantables ferromagnétiques mis en œuvre dans le procédé selon l'invention sont des gels possédant les propriétés des gels « aspirables », c'est-à-dire qu'en séchant, ils se fracturent et forment des déchets secs et solides facilement récupérables par brossage ou aspiration.

On démontre dans cet exemple que les déchets secs et solides obtenus par séchage des gels ferromagnétiques mis en œuvre dans le procédé selon l'invention peuvent également être récupérés à distance, par aimantation.

Les essais de cet exemple ont été réalisés dans une nacelle en acier inoxydable 316L.

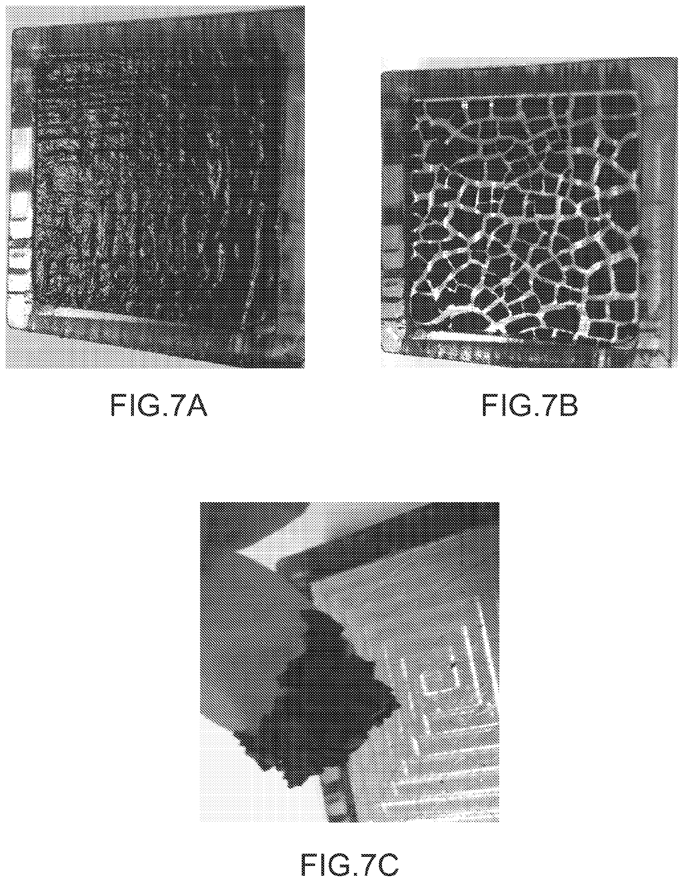

Les Figures 7A, 7B et 7C montrent la formation de déchets solides après séchage (à 25°C et 40% d'humidité relative) (Figure 7B) d'une couche d'une épaisseur de 1 mm de Gel 13 (76,2% de solution d'acide nitrique et 23,8% de phase solide, voir exemple 1) ainsi que la récupération de ces déchets à l'aide d'un aimant (Figure 7C).

On observe sur la Figure7A et la Figure 7B, que le séchage d'une couche de Gel 13 engendre bien la formation d'un déchet solide millimétrique. 2 g de gel ont été étalés (Figure 7A, avant séchage). Après séchage complet de la couche de gel (Figure 7B), la perte de masse est estimée à environ 1,5 g. Ainsi, environ 75% de la masse du gel se sont évaporés, soit la quasi-totalité de la phase liquide.

Sur la Figure 7C, un aimant (protégé par un plastique bleu) est utilisé pour récupérer les déchets solides après séchage. On observe visuellement que la totalité du déchet a pu être récupérée par aimantation. Après passage de l'aimant, on estime, par perte de masse, la quantité résiduelle de déchet qui reste adhéré à environ 0,08 g (soit 4%). Ce déchet est issu de couches très fines présentes sur les bords de la nacelle. Ainsi, une épaisseur suffisante de gel est nécessaire afin que les déchets engendrés puissent facilement se détacher du support et être attirés par aimantation.

Exemple 5 : Action de corrosion d'un gel aimantable ferromagnétique mis en œuvre dans le procédé selon l'invention sur de l'acier.

Dans cet exemple, l'action de corrosion d'un gel ferromagnétique mis en œuvre dans le procédé selon l'invention sur une surface est démontrée.

Le Gel 13 (voir exemple 1) est utilisé pour corroder une surface en acier au carbone.

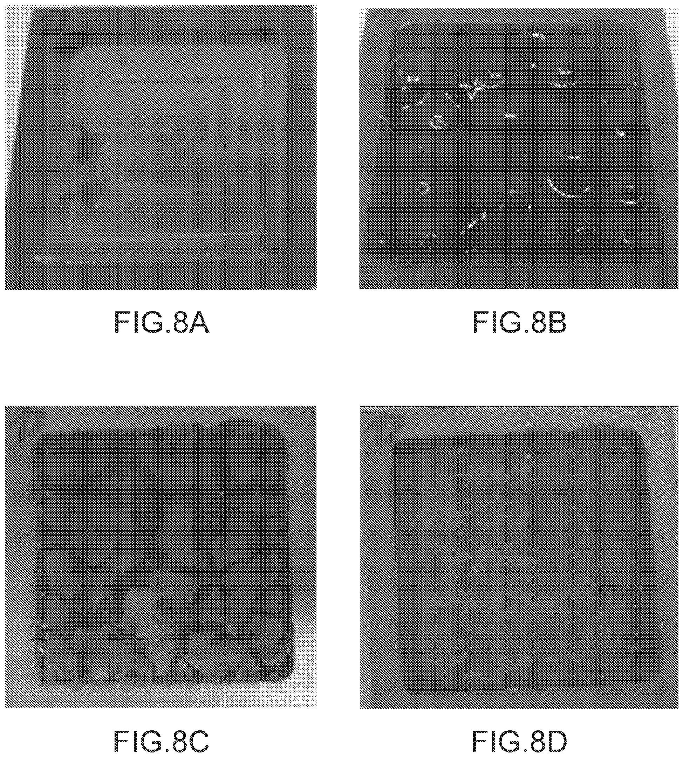

Une épaisseur de 1 mm de Gel 13 (comportant de l'acide nitrique à 17,5 %) est déposée dans une nacelle en acier au carbone préalablement pesée. Après séchage, les paillettes de gel sont éliminées et la nacelle est de nouveau pesée afin d'estimer sa perte de masse. Les Figures 8A, 8B, 8C, et 8D présentent les résultats obtenus.

On observe une nette dégradation de la surface après dépôt et séchage du gel (Figure 8D). Puis, une perte de masse de 0,5588 g est mesurée, ce qui démontre bien que les gels aimantables peuvent corroder des surfaces à la manière des gels aspirables, et qu'ils peuvent donc être utilisés pour la décontamination des surfaces.

L'épaisseur de corrosion est estimée comme suit :
- La surface totale recouverte par le gel (fond et bords de la nacelle) est de 23,85 cm².
- La masse volumique de l'acier utilisé est de 7,8 g/cm³.
- L'épaisseur corrodée est calculée comme étant égale au rapport masse / (surface x masse volumique).

On obtient ainsi une épaisseur corrodée moyenne d'environ 30 µm.

Exemple 6 : Décontamination d'un substrat à l'aide des gels aimantables ferromagnétiques mis en œuvre dans le procédé selon l'invention.

Dans cet exemple, les propriétés de décontamination radioactive des gels aimantables ferromagnétiques mis en œuvre dans le procédé selon invention sont démontrées.

Une solution de nitrate de césium est préparée, et sa concentration est déterminée comme étant égale à 2,86 mg.L⁻¹ par spectroscopie d'absorption atomique. Puis, 2 mL de cette solution sont déposés au fond d'une nacelle en acier inoxydable. On laisse ensuite la solution s'évaporer. On simule de cette manière une surface contaminée par 5,72 mg de ¹³³Cs (pouvant simuler une contamination radioactive en ¹³⁷Cs).

On dépose sur la surface contaminée artificiellement une couche de 1 mm de Gel 13 (voir exemple 1) et on laisse sécher le gel afin d'observer la formation des résidus solides.

Après séchage, les résidus solides sont récupérés et la surface est rincée à l'eau. L'eau de rinçage est analysée par spectroscopie d'absorption atomique afin de déterminer la quantité de Cs qui est restée adhérée au support et, par conséquent, l'efficacité du gel aimantable.

Après analyse, 0,58 mg de Cs ont été mesurés dans l'eau de rinçage de la surface. On peut alors calculer un Facteur de Décontamination (FD), que l'on définit comme FD = Masse de contamination initiale / masse de contamination finale. On obtient ainsi un FD d'environ 10 pour cet essai ce qui démontre bien l'efficacité de décontamination radioactive d'une surface par un gel aimantable ferromagnétique mis en œuvre dans le procédé selon l'invention.

### REFERENCES

[1] FAURE S., FUENTES P., LALLOT Y. *« Gel aspirable pour la décontamination de surfaces et utilisation »* : WO-A2-2007/039598.
[2] GOSSARD A., FRANCES F., LEPEYTRE C., « Gel de décontamination adsorbant et photocatalytique et procédé de décontamination de surface utilisant ce gel » : WO-A1-2018/011525.
[3] LUDWIG A., GOETTMANN F., FRANCES F., LEGOFF C., TANCHOU V., « Gel alcalin oxydant de décontamination biologique et procédé de décontamination biologique de surface utilisant ce gel » : WO-A1-2014/154818.
[4] GOSSARD A., FRANCES F., « Gel aspirable et procédé pour éliminer une contamination contenue dans une couche organique en surface d'un substrat solide » : WO-A1-2018/024990.
[5] GOSSARD A., TURC H.A., VENDITTI P., GRANDJEAN A., « Procédé de décontamination d'un milieu gazeux contaminé par des espèces contaminantes en suspension » : FR-A1-3083712.
[6] WO-A1-2014/154817.
[7] WO-A1-2013/092633.

## Revendications

1. Procédé de décontamination d'au moins une surface d'un substrat en un matériau solide, ladite surface étant contaminée par au moins une espèce contaminante se trouvant sur ladite surface et/ou sous ladite surface dans les premières couches du substrat, dans lequel on réalise au moins un cycle comprenant les étapes successives suivantes :
a) on applique un gel ferromagnétique inorganique constitué par une solution colloïdale comprenant un agent viscosant inorganique, un composé ferromagnétique, et un solvant, sur ladite surface, puis on déplace et on étale à distance le gel appliqué sur la surface à l'aide d'un aimant,
ledit agent viscosant inorganique étant, de préférence, choisi parmi les oxydes de métaux tels que les alumines, les oxydes de metalloïdes tels que les silices, les hydroxydes de métaux, les hydroxydes de metalloïdes, les oxyhydroxydes de métaux, les oxyhydroxydes de métalloïdes, les aluminosilicates, les argiles telles que la smectite, et leurs mélanges
ladite application étant réalisée, de préférence, par pulvérisation, au pinceau ou avec une taloche ;
b) on maintient le gel sur la surface au moins pendant une durée suffisante pour que le gel détruise et/ou inactive et/ou dégrade et/ou absorbe l'espèce contaminante, et pour que le gel sèche et forme sur la surface un résidu sec et solide contenant ladite espèce contaminante ;
c) on déplace et on rassemble sur la surface, à l'aide d'un aimant, le résidu sec et solide contenant ladite espèce contaminante, et on récupère le résidu sec et solide contenant ladite espèce contaminante,
le résidu sec et solide étant, de préférence, récupéré à l'aide d'un aimant et/ou par brossage et/ou aspiration, par exemple avec un aspirateur muni d'un aimant.

2. Procédé selon la revendication 1, dans lequel le substrat en un matériau solide est en un matériau choisi parmi les métaux et les alliages métalliques comme l'acier inoxydable, les aciers peints, l'aluminium et le plomb ; les polymères tels que les matières plastiques ou les caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments et les matériaux cimentaires; les mortiers et bétons ; les plâtres ; les briques ; la pierre naturelle ou artificielle ; les céramiques.

3. Procédé selon la revendication 1 ou 2, dans lequel le gel est appliqué sur la surface à décontaminer à raison de 100 g à 2000 g de gel par m² de surface, de préférence de 500 à 1500 g de gel par m² de surface, de préférence encore de 600 à 1000 g de gel par m² de surface, ce qui correspond généralement à une épaisseur de gel déposé sur la surface comprise entre 0,5 mm et 2 mm.

4. Procédé selon la revendication 1 ou 2, dans lequel, lors de l'étape b), une épaisseur de gel de 2 mm à 2 cm est maintenue sur la surface à l'aide d'un aimant.

5. Procédé selon l'une quelconque des revendications 1 à 4, dans lequel la surface est une surface interne d'un tuyau ou d'une canalisation, le gel est déposé sur la surface interne à l'entrée du tuyau ou de la canalisation, et est ensuite déplacé et étalé sur la surface à l'aide d'un aimant placé au voisinage de la surface externe ou sur la surface externe du tuyau ou de la canalisation.

6. Procédé de décontamination d'un volume d'un milieu gazeux contaminé par des espèces contaminantes en suspension, ledit volume d'un milieu gazeux étant en contact avec au moins une surface d'un substrat solide, ledit procédé comprenant les étapes successives suivantes :
a) on pulvérise dans ledit volume d'un milieu gazeux de fines gouttelettes, formant ainsi un brouillard, d'un gel ferromagnétique inorganique constitué par une solution colloïdale comprenant un agent viscosant inorganique, un composé ferromagnétique et un solvant,
ledit agent viscosant inorganique étant, de préférence, choisi parmi les oxydes de métaux tels que les alumines, les oxydes de metalloïdes tels que les silices, les hydroxydes de métaux, les hydroxydes de metalloïdes, les oxyhydroxydes de métaux, les oxyhydroxydes de métalloïdes, les aluminosilicates, les argiles telles que la smectite, et leurs mélanges ;
b) les espèces contaminantes en suspension sont capturées, captées par lesdites gouttelettes du gel ferromagnétique ;
c) les gouttelettes du gel ferromagnétique contenant les espèces contaminantes en suspension capturées sont déplacées sous l'action d'un aimant jusqu'à ce qu'elles se déposent et s'accumulent sur une zone déterminée de ladite surface du substrat solide ;
d) on maintient le gel sur la zone déterminée de la surface du substrat solide au moins pendant une durée suffisante pour que le gel sèche et forme un résidu sec et solide contenant les espèces contaminantes en suspension capturées ;
e) on récupère le résidu sec et solide contenant lesdites espèces contaminantes en suspension capturées, le résidu sec et solide étant, de préférence, récupéré à l'aide d'un aimant et/ou par brossage et/ou aspiration, par exemple avec un aspirateur muni d'un aimant.

7. Procédé selon la revendication 6, dans lequel le volume d'un milieu gazeux est un volume clos défini par des parois, telles qu'un sol, un plafond et des murs, formant ladite surface, le brouillard de fines gouttelettes remplit la totalité du volume clos, et les fines gouttelettes du gel ferromagnétique contenant les espèces contaminantes en suspension capturées se déposent sur au moins l'une des parois, de préférence sur une paroi inférieure telle qu'un sol.

8. Procédé selon la revendication 6 ou 7, dans lequel les fines gouttelettes ont une taille, définie par leur plus grande dimension, telle qu'un diamètre, de 1 à 1000 µm, de préférence de 5 à 200 µm.

9. Procédé selon l'une quelconque des revendications 6 à 8, dans lequel lesdites espèces contaminantes en suspension se présentent sous la forme de particules solides, de particules liquides, ou sous la forme d'espèces moléculaires.

10. Procédé selon l'une quelconque des revendications 1 à 9, dans lequel l'espèce contaminante est choisie, ou les espèces contaminantes sont choisies, parmi les espèces contaminantes ioniques, chimiques, biologiques, nucléaires ou radioactives.

11. Procédé selon l'une quelconque des revendications 1 à 10, dans lequel l'espèce contaminante est une espèce contaminante, ou les espèces contaminantes sont des espèces contaminantes, radioactive/s et/ou chimiquement toxique/s du fait de sa/leur forme et/ou de sa/leur taille, l'espèce contaminante, ou les espèces contaminantes, toxique/s du fait de sa/leur forme et/ou de sa/leur taille, étant de préférence choisie/s parmi les espèces contaminantes se présentant sous la forme de particules solides telles que des microparticules, ou des nanoparticules, par exemple sous la forme de fibres telles que des microfibres ou des nanofibres, sous la forme de nanotubes, ou sous la forme de cristaux tels que des nanocristaux.

12. Procédé selon l'une quelconque des revendications 1 à 11, dans lequel l'espèce contaminante, ou les espèces contaminantes, est/sont choisie/s parmi les métaux et métalloïdes sous forme de métal, de métalloïde, ou ionique, de préférence parmi les métaux dits « métaux lourds », et les métaux et métalloïdes toxiques sous forme de métal, de métalloïde, ou ionique; les composés de ces métaux et métalloïdes tels que les composés organométalliques, les sels de métaux, les oxydes de métaux, les carbures de métaux, etc. ; les céramiques ; le bois ; les céréales ; la farine ; l'amiante ; et les verres, par exemple sous la forme de laine de verre.

13. Procédé selon l'une quelconque des revendications précédentes, dans lequel la solution colloïdale comprend, en outre, un ou plusieurs composant/s choisi/s parmi les composants suivants :
- un agent tensioactif ;
- un agent actif de décontamination ;
- un agent piégeur pour piéger les espèces contaminantes gazeuses, notamment les espèces contaminantes gazeuses toxiques ou explosives, telles que l'hydrogène ;
- un polymère superabsorbant ;
- un agent extractant des espèces contaminantes.

14. Procédé selon l'une quelconque des revendications précédentes, dans lequel la solution colloïdale, comprend, de préférence est constituée par :
- 1% à 40% en masse, de préférence 5% à 30% en masse, de préférence encore 5% à 25% en masse, mieux 8% à 20% en masse, par rapport à la masse du gel, d'au moins un agent viscosant inorganique ;
- 0,1% à 40% en masse, de préférence 5 à 30 % en masse, par rapport à la masse du gel d'au moins un composé ferromagnétique ;
- éventuellement, 0,05% à 2% en masse par rapport à la masse du gel, d'au moins un agent tensioactif ;
- éventuellement 0,05 à 10 mol/L de gel, de préférence 0,1 à 5 mol/L de gel, de préférence encore 1 à 2 mol/L de gel, d'au moins un agent actif de décontamination ;
- éventuellement, 0,1% à 5% en masse, par rapport à la masse du gel, d'au moins un agent piégeur pour capter, piéger les espèces contaminantes gazeuses, notamment les espèces contaminantes gazeuses toxiques ou explosives, telles que l'hydrogène;
- éventuellement 0,05% à 5% en masse, de préférence 0,05% à 2% en masse, par rapport à la masse du gel, d'au moins un polymère superabsorbant ;
- éventuellement, 0,1% à 5% en masse, par rapport à la masse du gel, d'au moins un agent extractant des espèces contaminantes;
- et le reste de solvant, la quantité de solvant étant au minimum de 20% en masse, par rapport à la masse du gel.

15. Procédé selon la revendication 14, dans lequel l'agent tensioactif est choisi parmi les agents tensioactifs possédant une ou plusieurs parmi les propriétés mouillantes, les propriétés émulsifiantes et les propriétés détergentes ; et leurs mélanges et/ou
l'agent tensioactif est choisi dans le groupe constitué par les alcoxylates d'alcool, les alkyl-aryl sulphonates, les alkylphénol éthoxylates, les copolymères blocs à base d'oxyde d'éthylène et/ou d'oxyde de propylène, les alcools éthoxylés, les phosphates d'éther, les acides éthoxylés, les esters de glycérol, les imidazolines, les sels d'ammonium quaternaires (quats), les alcanolamides, les oxydes d'amines, et les mélanges de ceux-ci, et/ou
le composé ferromagnétique est choisi parmi les métaux ferromagnétiques, comme le fer, le cobalt et le nickel ; les alliages ferromagnétiques comme les alliages de Heusler, et les alliages formant les aimants permanents tels que les aimants permanents aux terres rares comme le néodyme ou le dysprosium ou au cobalt ; et les ferrites, et/ou
l'agent actif de décontamination est choisi parmi les bases telles que l'hydroxyde de sodium, l'hydroxyde de potassium, et leurs mélanges ; les acides tels que l'acide nitrique, l'acide phosphorique, l'acide chlorhydrique, l'acide sulfurique, les hydrogénooxalates comme l'hydrogénooxalate de sodium, et leurs mélanges ; les agents oxydants tels que les peroxydes, les permanganates, les persulfates, l'ozone, les hypochlorites tels que l'hypochlorite de sodium, les sels de cérium IV, et leurs mélanges; les sels d'ammonium quaternaires tels que les sels d'hexadécylpyridinium (cétylpyridinium) , comme le chlorure d'hexadécylpyridinium (cétylpyridinium); les agents réducteurs; et leurs mélanges, et/ou
le solvant est choisi parmi l'eau ; les solvants organiques tels que les terpènes et les alcools ; et leurs mélanges.

## Patentansprüche

1. Verfahren zur Dekontaminierung mindestens einer Oberfläche eines Substrats aus einem festen Material, wobei die Oberfläche mit mindestens einer kontaminierenden Spezies kontaminiert ist, die sich auf der Oberfläche und/oder unter der Oberfläche in den ersten Schichten des Substrats befindet, wobei mindestens ein Zyklus durchgeführt wird, der die folgenden aufeinanderfolgenden Schritte umfasst:
a) Aufbringen eines anorganischen ferromagnetischen Gels, das aus einer kolloidalen Lösung besteht, die ein anorganisches Viskositätsmittel, eine ferromagnetische Verbindung und ein Lösungsmittel umfasst, auf die Oberfläche, dann Bewegen und Verteilen des auf die Oberfläche aufgetragenen Gels mit Hilfe eines Magneten in einem gewissen Abstand,
wobei das anorganische Viskositätsmittel vorzugsweise unter den Metalloxiden wie Aluminiumoxiden, Metalloidoxiden wie Siliciumdioxid, Metallhydroxiden, Metalloidhydroxiden, Metalloxyhydroxiden, Metalloidoxyhydroxiden, Aluminosilikaten, Tonen wie Smektit und Mischungen davon ausgewählt wird,
wobei die Anwendung vorzugsweise durch Aufsprühen, mit einem Pinsel oder mit einer Glättkelle erfolgt;
b) Halten des Gels auf der Oberfläche für mindestens einen Zeitraum, der ausreicht, damit das Gel die kontaminierende Spezies zerstört und/oder inaktiviert und/oder abbaut und/oder absorbiert, und damit das Gel trocknet und auf der Oberfläche einen trockenen, festen Rückstand bildet, der die kontaminierende Spezies enthält;
c) Bewegen und Sammeln des trockenen und festen Rückstands, der die kontaminierende Spezies enthält, auf der Oberfläche mit Hilfe eines Magneten und Sammeln des trockenen und festen Rückstands, der die kontaminierende Spezies enthält,
wobei der trockene und feste Rückstand vorzugsweise mit Hilfe eines Magneten und/oder durch Bürsten und/oder Saugen, beispielsweise mit einem mit einem Magneten versehenen Staubsauger, gewonnen wird.

2. Verfahren nach Anspruch 1, wobei das Substrat aus einem festen Material besteht, das aus Metallen und Metalllegierungen wie rostfreiem Stahl, lackiertem Stahl, Aluminium und Blei ausgewählt ist; Polymeren wie Kunststoffen oder Kautschuken wie Poly(vinylchlorid) oder PVC, Polypropylen oder PP, Polyethylen oder PE, insbesondere Polyethylen hoher Dichte oder HDPE, Poly(methylmethacrylat) oder PMMA, Poly(vinylidenfluorid) oder PVDF, Polycarbonaten oder PC; Glas; Zement und zementhaltigen Materialien; Mörtel und Beton; Gips; Ziegel; Natur- und Kunststein; Keramik.

3. Verfahren nach Anspruch 1 oder 2, wobei das Gel auf die zu dekontaminierende Oberfläche in einer Menge von 100 g bis 2000 g Gel pro m² Oberfläche, vorzugsweise 500 bis 1500 g Gel pro m² Oberfläche, noch bevorzugter 600 bis 1000 g Gel pro m² Oberfläche, aufgetragen wird, was im Allgemeinen einer Dicke des auf der Oberfläche abgeschiedenen Gels zwischen 0,5 mm und 2 mm entspricht.

4. Verfahren nach Anspruch 1 oder 2, wobei in Schritt b) eine Gelschichtdicke von 2 mm bis 2 cm mit Hilfe eines Magneten auf der Oberfläche gehalten wird.

5. Verfahren nach einem der Ansprüche 1 bis 4, wobei die Oberfläche eine innere Oberfläche eines Rohrs oder einer Leitung ist, das Gel auf die innere Oberfläche am Eingang des Rohrs oder der Leitung aufgebracht wird und dann mit Hilfe eines Magneten, der in der Nähe der äußeren Oberfläche oder auf der äußeren Oberfläche des Rohrs oder der Leitung angeordnet ist, über die Oberfläche bewegt und verteilt wird.

6. Verfahren zur Dekontaminierung eines Volumens eines gasförmigen Mediums, das mit suspendierten kontaminierenden Spezies kontaminiert ist, wobei das Volumen eines gasförmigen Mediums mit mindestens einer Oberfläche eines festen Substrats in Kontakt steht, wobei das Verfahren die folgenden aufeinanderfolgenden Schritte umfasst:
a) Sprühen von feinen Tröpfchen eines anorganischen ferromagnetischen Gels, das aus einer kolloidalen Lösung besteht, die ein anorganisches Viskositätsmittel, eine ferromagnetische Verbindung und ein Lösungsmittel umfasst, in das Volumen eines gasförmigen Mediums, wodurch ein Nebel gebildet wird,
wobei das anorganische Viskositätsmittel vorzugsweise ausgewählt ist aus Metalloxiden wie Aluminiumoxiden, Metalloidoxiden wie Siliciumdioxid, Metallhydroxiden, Metalloidhydroxiden, Metalloxyhydroxiden, Metalloidoxyhydroxiden, Aluminosilikaten, Tonen wie Smektit und Mischungen davon;
b) Einfangen der schwebenden kontaminierenden Spezies, die von den Tröpfchen des ferromagnetischen Gels eingefangen werden;
c) Bewegen der Tröpfchen des ferromagnetischen Gels, die die eingefangenen suspendierten kontaminierenden Spezies enthalten, unter der Wirkung eines Magneten, bis sie sich auf einer bestimmten Zone der Oberfläche des festen Substrats absetzen und ansammeln;
d) Halten des Gels auf der bestimmten Zone der Oberfläche des festen Substrats mindestens so lange, dass das Gel trocknet und einen trockenen, festen Rückstand bildet, der die eingefangenen suspendierten kontaminierenden Spezies enthält;
e) Gewinnen des trockenen und festen Rückstands, der die eingefangenen suspendierten kontaminierenden Spezies enthält, wobei der trockene und feste Rückstand vorzugsweise mit Hilfe eines Magneten und/oder durch Bürsten und/oder Saugen, zum Beispiel mit einem mit einem Magneten versehenen Staubsauger, gewonnen wird.

7. Verfahren nach Anspruch 6, wobei das Volumen eines gasförmigen Mediums ein geschlossenes Volumen ist, das durch Wände wie Boden, Decke und Wände definiert ist, die die Oberfläche bilden, der Nebel aus feinen Tröpfchen das gesamte geschlossene Volumen ausfüllt und die feinen Tröpfchen des ferromagnetischen Gels, das die eingefangenen suspendierten kontaminierenden Spezies enthält, sich an mindestens einer der Wände, vorzugsweise an einer unteren Wand wie einem Boden, absetzen.

8. Verfahren nach Anspruch 6 oder 7, wobei die feinen Tröpfchen eine Größe, definiert durch ihre größte Abmessung, wie einen Durchmesser, von 1 bis 1000 µm, vorzugsweise 5 bis 200 µm, aufweisen.

9. Verfahren nach einem der Ansprüche 6 bis 8, wobei die suspendierten kontaminierenden Spezies in Form von festen Partikeln, flüssigen Partikeln oder in Form von molekularen Spezies vorliegen.

10. Verfahren nach einem der Ansprüche 1 bis 9, wobei die kontaminierende Spezies aus ionischen, chemischen, biologischen, nuklearen oder radioaktiven kontaminierenden Spezies ausgewählt wird oder ausgewählt werden.

11. Verfahren nach einem der Ansprüche 1 bis 10, wobei die kontaminierende Spezies eine kontaminierende Spezies ist oder die kontaminierenden Spezies kontaminierende Spezies sind, die aufgrund ihrer Form und/oder Größe radioaktiv und/oder chemisch toxisch ist/sind, und wobei die kontaminierende Spezies oder die kontaminierenden Spezies aufgrund ihrer Form und/oder Größe toxisch ist/sind, vorzugsweise ausgewählt aus kontaminierenden Spezies in Form von festen Partikeln wie Mikropartikeln oder Nanopartikeln, zum Beispiel in Form von Fasern wie Mikrofasern oder Nanofasern, in Form von Nanoröhren oder in Form von Kristallen wie Nanokristallen.

12. Verfahren nach einem der Ansprüche 1 bis 11, wobei die kontaminierende Spezies oder die kontaminierenden Spezies ausgewählt ist/sind aus Metallen und Metalloiden in Form von Metall, Metalloid oder Ionen, vorzugsweise aus den sogenannten "Schwermetallen", und toxischen Metallen und Metalloiden in Form von Metall, Metalloid oder Ionen; Verbindungen dieser Metalle und Metalloide wie metallorganische Verbindungen, Metallsalze, Metalloxide, Metallcarbide usw.; Keramik; Holz; Getreide; Mehl; Asbest; und Glas, zum Beispiel in Form von Glaswolle.

13. Verfahren nach einem der vorstehenden Ansprüche, wobei die kolloidale Lösung ferner eine oder mehrere Komponenten umfasst, die aus den folgenden Komponenten ausgewählt sind:
- einem oberflächenaktiven Mittel;
- einem aktiven Dekontaminationsmittel;
- einem Einfangmittel zum Einfangen gasförmiger kontaminierender Spezies, insbesondere toxischer oder explosiver gasförmiger kontaminierender Spezies, wie beispielsweise Wasserstoff;
- einem superabsorbierenden Polymer;
- ein Extraktionsmittel für kontaminierende Spezies.

14. Verfahren nach einem der vorstehenden Ansprüche, wobei die kolloidale Lösung Folgendes umfasst, vorzugsweise bestehend aus:
- 1 bis 40 Massen-%, vorzugsweise 5 bis 30 Massen-%, besonders bevorzugt 5 bis 25 Massen-%, besonders bevorzugt 8 bis 20 Massen-%, bezogen auf die Masse des Gels, mindestens eines anorganischen Viskositätsmittels;
- 0,1 bis 40 Massen-%, vorzugsweise 5 bis 30 Massen-%, bezogen auf die Masse des Gels, mindestens einer ferromagnetischen Verbindung;
- gegebenenfalls 0,05 bis 2 Massen-%, bezogen auf die Masse des Gels, mindestens eines oberflächenaktiven Mittels;
- gegebenenfalls 0,05 bis 10 mol/L Gel, vorzugsweise 0,1 bis 5 mol/L Gel, noch bevorzugter 1 bis 2 mol/L Gel, mindestens eines aktiven Dekontaminationsmittels;
- gegebenenfalls 0,1 bis 5 Massen-%, bezogen auf die Masse des Gels, mindestens eines Einfangmittels zum Auffangen, Einfangen von gasförmigen kontaminierenden Spezies, insbesondere von toxischen oder explosiven gasförmigen kontaminierenden Spezies wie Wasserstoff;
- gegebenenfalls 0,05 Massen-% bis 5 Massen-%, vorzugsweise 0,05 Massen-% bis 2 Massen-%, bezogen auf die Masse des Gels, mindestens eines superabsorbierenden Polymers;
- gegebenenfalls 0,1 Massen-% bis 5 Massen-%, bezogen auf die Masse des Gels, mindestens eines Extraktionsmittels für kontaminierende Spezies;
- und dem Rest des Lösungsmittels, wobei die Menge des Lösungsmittels mindestens 20 -%, bezogen auf die Masse des Gels, beträgt.

15. Verfahren nach Anspruch 14, wobei das Tensid ausgewählt ist aus Tensiden mit einer oder mehreren Benetzungseigenschaften, Emulgiereigenschaften und Detergenzeigenschaften; und Mischungen davon und/oder
das Tensid aus der Gruppe ausgewählt ist, bestehend aus Alkoholalkoxylaten, Alkylarylsulfonaten, Alkylphenolethoxylaten, Blockcopolymeren auf der Basis von Ethylenoxid und/oder Propylenoxid, ethoxylierten Alkoholen, Etherphosphaten, ethoxylierten Säuren, Glycerinestern, Imidazolinen, quaternären Ammoniumsalzen (Quats), Alkanolamiden, Aminoxiden und Mischungen davon, und/oder
die ferromagnetische Verbindung ausgewählt ist aus ferromagnetischen Metallen wie Eisen, Kobalt und Nickel; ferromagnetischen Legierungen wie Heusler-Legierungen und Legierungen, die Permanentmagnete bilden, wie Permanentmagnete aus seltenen Erden wie Neodym oder Dysprosium oder aus Kobalt; und Ferriten, und/oder
das aktive Dekontaminationsmittel ausgewählt ist aus Basen wie Natriumhydroxid, Kaliumhydroxid und deren Mischungen; Säuren wie Salpetersäure, Phosphorsäure, Salzsäure, Schwefelsäure, Hydrogenoxalate wie Natriumhydrogenoxalat und deren Mischungen; Oxidationsmittel wie Peroxide, Permanganate, Persulfate, Ozon, Hypochlorite wie Natriumhypochlorit, Cer-IV-Salze und Mischungen davon; quaternäre Ammoniumsalze wie Hexadecylpyridinium-(Cetylpyridinium)-Salze wie Hexadecylpyridinium-(Cetylpyridinium)-Chlorid; Reduktionsmittel und Mischungen davon, und/oder
das Lösungsmittel ausgewählt ist aus Wasser; organischen Lösungsmitteln wie Terpenen und Alkoholen; und Mischungen davon.

## Claims

1. A method for decontaminating at least one surface of a substrate of a solid material, said surface being contaminated with at least one contaminant species located on said surface and/or below said surface in the first layers of the substrate, wherein at least one cycle comprising the following successive steps is performed:
a) an inorganic ferromagnetic gel consisting of a colloidal solution comprising an inorganic viscosifier, a ferromagnetic compound and a solvent is applied to said surface, and then the gel applied to the surface is moved and spread at a distance using a magnet,
said inorganic viscosifier being preferably selected from metal oxides such as aluminas, metalloid oxides such as silicas, metal hydroxides, metalloid hydroxides, metal oxyhydroxides, metalloid oxyhydroxides, aluminosilicates, clays such as smectite, and mixtures thereof,
said application being preferably performed by spraying, brushing or trowelling;
b) the gel is maintained on the surface at least for a time sufficient for the gel to destroy and/or inactivate and/or degrade and/or absorb the contaminant species, and for the gel to dry and form a dry, solid residue containing said contaminant species on the surface;
c) the dry, solid residue containing said contaminant species is moved and gathered on the surface using a magnet, and the dry, solid residue containing said contaminant species is recovered,
the dry, solid residue being preferably recovered using a magnet and/or by brushing and/or suction, for example with a suction device provided with a magnet.

2. The method according to claim 1, wherein the substrate of a solid material is of a material selected from metals and metal alloys such as stainless steel, painted steels, aluminium and lead; polymers such as plastic materials or rubbers such as polyvinyl chlorides (PVC), polypropylenes (PP), polyethylenes (PE), in particular high density polyethylenes (HDPE), poly(methyl methacrylates) (PMMA), poly(vinylidene fluorides) (PVDF), polycarbonates (PC); glasses; cements and cementitious materials; mortars and concretes; plasters; bricks; natural or artificial stone; ceramics.

3. The method according to claim 1 or 2, wherein the gel is applied to the surface to be decontaminated at a rate of from 100 g to 2000 g of gel per m² of area, preferably from 500 to 1500 g of gel per m² of area, still more preferably from 600 to 1000 g of gel per m² of area, which generally corresponds to a thickness of gel deposited onto the surface of between 0.5 mm and 2 mm.

4. The method according to claim 1 or 2, wherein, during step b), a thickness of gel of from 2 mm to 2 cm is maintained on the surface using a magnet.

5. The method according to any one of claims 1 to 4, wherein the surface is an inner surface of a pipe or duct, the gel is deposited onto the inner surface at the inlet of the pipe or duct, and is then moved and spread on the surface by means of a magnet placed in the vicinity of the outer surface or on the outer surface of the pipe or duct.

6. A method for decontaminating a volume of a gaseous medium contaminated with suspended contaminant species, said volume of a gaseous medium being in contact with at least one surface of a solid substrate, said method comprising the following successive steps:
a) an inorganic ferromagnetic gel consisting of a colloidal solution comprising an inorganic viscosifier, a ferromagnetic compound and a solvent is sprayed into said volume of a gaseous medium of fine droplets, thereby forming a mist,
said inorganic viscosifier being preferably selected from metal oxides such as aluminas, metalloid oxides such as silicas, metal hydroxides, metalloid hydroxides, metal oxyhydroxides, metalloid oxyhydroxides, aluminosilicates, clays such as smectite, and mixtures thereof;
b) the suspended contaminant species are captured, taken up by said droplets of ferromagnetic gel;
c) the droplets of ferromagnetic gel containing the suspended contaminant species captured are moved under the action of a magnet until they are deposited and accumulate on a determined zone of said surface of the solid substrate;
d) the gel is maintained on the determined zone of the surface of the solid substrate at least for a time sufficient for the gel to dry and form a dry, solid residue containing the suspended contaminant species captured;
e) the dry, solid residue containing said suspended contaminant species captured is recovered, the dry, solid residue being preferably recovered using a magnet and/or by brushing and/or suction, for example with a suction device provided with a magnet.

7. The method according to claim 6, wherein the volume of a gaseous medium is an enclosed volume defined by partitions, such as a floor, a ceiling and walls, forming said surface, the mist of fine droplets fills the entire enclosed volume, and the fine droplets of ferromagnetic gel containing the suspended contaminant species captured are deposited onto at least one of the partitions, preferably on a lower partition such as a floor.

8. The method according to claim 6 or 7, wherein the fine droplets have a size, defined by their largest dimension, such as a diameter, of from 1 to 1000 µm, preferably from 5 to 200 µm.

9. The method according to any one of claims 6 to 8, wherein said suspended contaminant species are in the form of solid particles, liquid particles, or in the form of molecular species.

10. The method according to any one of claims 1 to 9, wherein the contaminant species is selected, or the contaminant species are selected, from ionic, chemical, biological, nuclear or radioactive contaminant species.

11. The method according to any one of claims 1 to 10, wherein the contaminant species is, or the contaminant species are, radioactive and/or chemically toxic and/or toxic contaminant species due to its/their shape and/or size, the contaminant species, or contaminant species, toxic due to its/their shape and/or size, being preferably selected from contaminant species in the form of solid particles such as microparticles, or nanoparticles, for example in the form of fibres such as microfibres or nanofibres, in the form of nanotubes, or in the form of crystals such as nanocrystals.

12. The method according to any one of claims 1 to 11, wherein the contaminant species, or the contaminant species, is/are selected from metals and metalloids in metal, metalloid or ionic form, preferably from so-called "heavy metals", and toxic metals and metalloids in metal, metalloid or ionic form; compounds of these metals and metalloids, such as organometallic compounds, metal salts, metal oxides, metal carbides, etc. ceramics; wood; cereals; flour; asbestos; and glasses, for example in the form of glass wool.

13. The method according to any one of the preceding claims, wherein the colloidal solution further comprises one or more component/s selected from the following components:
- a surfactant;
- an active decontamination agent;
- a getter for trapping gaseous contaminant species, in particular toxic or explosive gaseous contaminant species, such as hydrogen;
- a superabsorbent polymer;
- a contaminant species extractant.

14. The method according to any one of the preceding claims, wherein the colloidal solution comprises, preferably consists of:
- 1% to 40% by mass, preferably 5% to 30% by mass, more preferably 5% to 25% by mass, still more preferably 8% to 20% by mass, relative to the mass of the gel, of at least one inorganic viscosifier;
- 0.1% to 40% by mass, preferably 5% to 30% by mass, relative to the mass of the gel, of at least one ferromagnetic compound;
- optionally, 0.05% to 2% by mass, relative to the mass of the gel, of at least one surfactant;
- optionally, 0.05 to 10 mol/L of gel, preferably 0.1 to 5 mol/L of gel, even more preferably 1 to 2 mol/L of gel, of at least one active decontamination agent;
- optionally, 0.1% to 5% by mass, relative to the mass of the gel, of at least one getter to take up, trap gaseous contaminant species, in particular toxic or explosive gaseous contaminant species, such as hydrogen;
- optionally, 0.05% to 5% by mass, preferably 0.05% to 2% by mass, relative to the mass of the gel, of at least one superabsorbent polymer;
- optionally, 0.1% to 5% by mass, relative to the mass of the gel, of at least one contaminant species extractant;
- and the remainder solvent, the amount of solvent being at least 20% by mass, relative to the mass of the gel.

15. The method according to claim 14, wherein the surfactant is selected from surfactants having one or more of wetting properties, emulsifying properties and detergent properties; and mixtures thereof and/or
the surfactant is selected from the group consisting of alcohol alkoxylates, alkyl aryl sulphonates, alkyl phenol ethoxylates, block copolymers based on ethylene oxide and/or propylene oxide, ethoxylated alcohols, ether phosphates, ethoxylated acids, glycerol esters, imidazolines, quaternary ammonium salts (quats), alkanolamides, amine oxides, and mixtures thereof, and/or
the ferromagnetic compound is selected from ferromagnetic metals, such as iron, cobalt and nickel; ferromagnetic alloys such as Heusler alloys, and alloys forming permanent magnets such as rare earth permanent magnets such as neodymium or dysprosium or cobalt permanent magnets; and ferrites, and/or
the active decontamination agent is selected from bases such as sodium hydroxide, potassium hydroxide, and mixtures thereof; acids such as nitric acid, phosphoric acid, hydrochloric acid, sulphuric acid, hydrogen oxalates such as sodium hydrogen oxalate, and mixtures thereof; oxidising agents such as peroxides, permanganates, persulphates, ozone, hypochlorites such as sodium hypochlorite, cerium IV salts, and mixtures thereof; quaternary ammonium salts such as hexadecylpyridinium (cetylpyridinium) salts, such as hexadecylpyridinium (cetylpyridinium) chloride; reducing agents; and mixtures thereof, and/or
the solvent is selected from water; organic solvents such as terpenes and alcohols; and mixtures thereof.
